# EUROPÄISCHE PATENTSCHRIFT

(11) **EP 4 094 793 B1**
(45) Veröffentlichungstag und Bekanntmachung des Hinweises auf die Patenterteilung: **02.10.2024**
(21) Anmeldenummer: 22174918.7
(22) Anmeldetag: 23.05.2022
(51) Int. Cl.: A61M 11/00, B05B 17/00, A61M 16/00, B06B 1/06, A61M 16/14

(54) **VORRICHTUNG ZUR ERZEUGUNG VON AEOROSOLEN**
DEVICE FOR GENERATING AEROSOLS
DISPOSITIF DE GÉNÉRATION DES AÉROSOLS

(30) Priorität: 28.05.2021 DE 102021002767
(43) Veröffentlichungstag der Anmeldung: 30.11.2022
(73) Patentinhaber: Löwenstein Medical Technology S.A., 2557 Luxembourg (LU)
(72) Erfinder: Baumann, Josef, 94036 Passau (DE); Kremeier, Peter, 76149 Karlsruhe (DE)
(74) Vertreter: Marx, Thomas

(56) Entgegenhaltungen:
- US-A1- 2005 011 514
- US-A1- 2013 291 859
- US-A1- 2016 228 903
- US-A1- 2017 072 160

## Beschreibung

Die Erfindung betrifft eine Vorrichtung zur Erzeugung von Aerosolen sowie ein Verfahren und Bauteil zur Einstellung der Aerosolmenge.

Schwingmembranvernebler besitzen gegenüber anderen Vernebler-Technologien, wie Düsenverneblern oder Ultraschallverneblern, wesentliche Vorteile. So kann durch die Aerosolerzeugung mittels Mikrolöchern ein sehr feines Primäraerosol erzeugt werden. So entfällt weitestgehend, dass sich die größeren Tröpfchen abscheiden, bevor das Aerosol inhaliert wird. Dadurch kann ein größerer Anteil der eingefüllten Medikamentenmenge die tiefen Regionen der Lunge erreichen.

Die am Markt befindlichen Systeme haben die Einschränkung, dass der Ausstoß des Aerosols nicht eingestellt werden kann. Bei der Applikation von verschiedenen Medikamenten kann es von Vorteil sein, die Abgaberate auf die Dosierungsanforderung des Medikamentes einzustellen. Beispielsweise kann es von Vorteil sein, dass ein Medikament nicht zu schnell im Körper anflutet. Eine Anpassung der Aerosolabgaberate ist jedoch heute mit einer technischen Änderung verbunden (z.B. Reduzierung der Löcher in der Membran). Diese bedeutet einen enormen Entwicklungs- und Zulassungsaufwand und ist mit einer technischen Vervielfachung der Komponenten verbunden.

US 2005/0011514 A1 beschreibt einen Vernebler zum Verabreichen eines Medikaments. Der Vernebler umfasst ein Gehäuse mit einem Reservoir für das Medikament und einen Aerosolgenerator zum Umwandeln des Medikaments in ein Aerosol. Der Aerosolgenerator kann ein über einen Schwingkreis ansteuerbares Piezoelement umfassen.

US 2013/0291859 A1 beschreibt ein weiteres Beispiel für einen Vernebler mit einem Piezoelement.

Aufgabe der vorliegenden Erfindung ist es, ein System bereitzustellen, welches einfach in der Handhabung ist und ein breites Verwendungsspektrum abdeckt. Die Aufgabe wird gelöst durch einen Vernebler nach Anspruch 1, ein Verfahren nach Anspruch 12, und ein Beatmungssystem nach Anspruch 16.

In manchen Ausführungsformen ist der Vernebler auch dadurch gekennzeichnet, dass die Steuerelektronik dazu eingerichtet ist, eine Anregungsfrequenz für den Aerosolgenerator und/oder den Schwingkreis vorzugeben.

In manchen Ausführungsformen ist der Vernebler auch dadurch gekennzeichnet, dass die Steuerelektronik dazu eingerichtet ist, ein Sinus-förmiges Anregungssignal zu erzeugen.

In manchen Ausführungsformen ist der Vernebler auch dadurch gekennzeichnet, dass das erzeugte Anregungssignal eine höhere Spannung aufweist als das Rechtecksignal.

In manchen Ausführungsformen ist der Vernebler auch dadurch gekennzeichnet, dass der Vernebler so eingerichtet ist über die Pulsbreite die Menge an erzeugtem Aerosol zu steuern. In manchen Ausführungsformen ist der Vernebler auch dadurch gekennzeichnet, dass die Pulsbreite des Rechtecksignals maximal 50% erreicht.

In manchen Ausführungsformen ist der Vernebler auch dadurch gekennzeichnet, dass die Pulsbreite des Rechtecksignals maximal 30% erreicht.

In manchen Ausführungsformen ist der Vernebler auch dadurch gekennzeichnet, dass der HF-Generator zumindest einen Oszillator und einen Pulsweitenmodulator umfasst, wobei der Pulsweitenmodulator dazu eingerichtet ist, die Pulsweite des Rechtecksignals vorzugeben.

In manchen Ausführungsformen ist der Vernebler auch dadurch gekennzeichnet, dass die Steuerelektronik zumindest eine Einrichtung zum Messen einer Spannung umfasst, wobei die Einrichtung zum Messen der Spannung so angeordnet ist, dass die Spannung am Aerosolgenerator aufgenommen wird und die Steuerlektronik so eingerichtet ist, aus der Spannungsmessung die optimale Frequenz des Anregungssignals zu ermitteln.

In manchen Ausführungsformen ist der Vernebler auch dadurch gekennzeichnet, dass die Steuerelektronik dazu eingerichtet ist, die optimale Anregungsfrequenz über einen Spannungsabfall am Aerosolgenerator bei Überschreiten der optimalen Anregungsfrequenz zu ermitteln

In manchen Ausführungsformen ist der Vernebler auch dadurch gekennzeichnet, dass die optimale Anregungsfrequenz der Resonanzfrequenz des Aerosolgenerators und/oder des Schwingkreises entspricht.

In manchen Ausführungsformen ist der Vernebler auch dadurch gekennzeichnet, dass die Steuerelektronik dazu eingerichtet ist, die optimale Anregungsfrequenz und/oder Resonanzfrequenz des Aerosolgenerators und/oder Schwingkreises automatisch zu ermitteln.

In manchen Ausführungsformen ist der Vernebler auch dadurch gekennzeichnet, dass die Steuerelektronik dazu eingerichtet ist, die optimale Anregungsfrequenz regelmäßig zu überprüfen und automatisch anzupassen.

In manchen Ausführungsformen ist der Vernebler auch dadurch gekennzeichnet, dass der Vernebler mit einem Beatmungsgerät verbindbar ist und die Steuerelektronik dazu eingerichtet ist, Vorgaben zur Aerosolerzeugung vom Beatmungsgerät zu empfangen und umzusetzen.

In manchen Ausführungsformen ist der Vernebler auch dadurch gekennzeichnet, dass der Vernebler ein Verneblerteil und ein Elektronikteil umfasst.

In manchen Ausführungsformen ist der Vernebler auch dadurch gekennzeichnet, dass das Verneblerteil zumindest den Aerosolgenerator und das Flüssigkeitsreservoir umfasst, wobei das Flüssigkeitsreservoir so ausgebildet ist, dass eine Flüssigkeit an die Membran des Aerosolgenerators herangeführt wird.

In manchen Ausführungsformen ist der Vernebler auch dadurch gekennzeichnet, dass das Elektronikteil zumindest die Steuerelektronik umfasst.

In manchen Ausführungsformen ist der Vernebler auch dadurch gekennzeichnet, dass der Aerosolgenerator in dem Verneblerteil in einer Fassung gelagert wird, wobei die Fassung über zumindest eine Öffnung verfügt durch welche die Flüssigkeit an die Membran herangeführt wird und dass das Flüssigkeitsreservoir über eine Wand von einer Einführöffnung für das Elektronikteil getrennt ist, wobei die Wand mit dem Rand der Öffnung abschließt.

Beansprucht wird auch ein Verfahren zur Einstellung der Aerosolgeneration eines Verneblers, umfassend zumindest einen der folgenden Schritte:
- Finden der Resonanzfrequenz des Aerosolgenerators und/oder des Schwingkreises (44)
- Bestimmen des optimalen Wirkungsgrades
- Einstellen der Pulsweite
- Einstellen der Menge an erzeugtem/zu erzeugendem Aerosol.

In manchen Ausführungsformen ist das Verfahren auch dadurch gekennzeichnet, dass zum Finden der Resonanzfrequenz des Aerosolgenerators und/oder des Schwingkreises ein Bereich von Anregungsfrequenzen abgetastet wird und gleichzeitig die Spannung am Aerosolgenerator gemessen wird, wobei die Resonanzfrequenz erreicht ist, wenn eine Erhöhung der Anregungsfrequenz zu einem Abfall der gemessenen Spannung führt.

In manchen Ausführungsformen ist das Verfahren auch dadurch gekennzeichnet, dass das Einstellen der Menge an erzeugtem/zu erzeugendem Aerosol eine Variation der Pulsweite umfasst, wobei einer Verringerung der Pulsbreite unterhalb der Pulsbreite für den optimalen Wirkungsgrad eine Verringerung der erzeugten Aerosolmenge folgt.

Beansprucht wird auch ein Beatmungssystem umfassend zumindest ein Beatmungsgerät und einen erfindungsgemäßen Vernebler, wobei das Beatmungssystem zumindest eine Steuerelektronik zur Steuerung des Verneblers umfasst.

Das Beatmungssystem ist auch dadurch gekennzeichnet, dass die Steuerelektronik zumindest teilweise in dem Vernebler angeordnet ist.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface bzw. Patientenschnittstelle kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. Dazu können auch Highflow-Masken bzw. -Schnittstellen unter den Begriff des Patienteninterface fallen. In manchen Fällen kann das Patienteninterface auch ein einfaches Mundstück, beispielsweise ein Rohr, sein, durch welches das Lebewesen zumindest ausatmet und/oder einatmet

Es ist weiter anzumerken, dass der Begriff der Menge an erzeugtem Aerosol sich, soweit nicht ausdrücklich anders beschrieben, auf die erzeugte Menge an Aerosol pro Periode der Membranbewegung bezogen ist. Der Begriff Pulsweite wird synonym für den Begriff Pulsbreite genutzt, beide Begriffe bezeichnen das Gleiche. Auch kann der Begriff Pulsdauer als Synonym gesehen werden.

Insbesondere betrifft die Erfindung einen Vernebler sowie ein Verfahren zur Steuerung eines Verneblers. Besonders vorteilhaft ist dabei, dass die Menge an erzeugten Aerosol durch den Vernebler einfach und effizient anpassbar ist, ohne Bauteile des Verneblers zu tauschen.

Das Schwingsystem, zum Beispiel der Schwingkreis bestehend aus dem Aerosolgenerator und der Induktivität, wird durch ein periodisches Signal angeregt, welches in einer elektronischen Schaltung, zum Beispiel der Steuerelektronik, erzeugt wird. Gemäß der Erfindung wird ein vorteilhafter Aufbau der Steuerelektronik vorgestellt, über welche die Aerosolerzeugung auf einfach Weise beeinflusst und optimiert werden kann. Beispielsweise kann erreicht werden, dass die Anregungsfrequenz so festgelegt werden kann, dass eine hohe Aerosolerzeugungsrate bzw. -menge bei geringem Leistungsbedarf erzielt werden kann. Damit zusammenhängend kann durch eine einfache Regelung auch der Wirkungsgrad optimiert werden.

Insbesondere kann die Aerosolerzeugung allein über Eingaben in einer Software in einem weiten Bereich einstellbar sein. Die Software ist beispielsweise so eingerichtet, dass die Vorgaben über eine Steuereinheit an die Steuerelektronik weitergegeben werden. Insbesondere über diese Vorgaben über eine Software und/oder Steuereinheit, beispielsweise in entsprechend eingerichteten Beatmungsgeräten, sind unterschiedlichste Applikationsformen, Produktvarianten und/oder Einsatzgebiete realisierbar, ohne das die Hardware, wie zum Beispiel die verbaute Membran, geändert werden muss.

In manchen Ausführungsformen ist der Vernebler zumindest zweiteilig ausgeführt und umfasst einen Verneblerteil und einen Elektronikteil. Der Verneblerteil kann beispielsweise als Einweg-Produkt ausgebildet sein und nur mit einem Patienten über eine gewisse Zeitdauer verwendet und danach entsorgt werden. Beispielsweise sind daher nur die unbedingt notwendigen Komponenten wie der Aerosolgenerator und das Flüssigkeitsreservoir verbaut. Die Einzelteile des Verneblerteils sind beispielsweise über einfache Fügeschritte (Positionieren, dann Verpressen und/oder Einrasten) zu montieren.

Das Elektronikteil hingegen ist in der Regel wiederverwendbar und kann für mehrere Patienten verwendet werden. In manchen Ausführungsformen umfasst das Elektronikteil zumindest teilweise die Steuerelektronik. Da die Elektronik in das Verbindungsteil integriert ist, entfallen zusätzliche Komponenten wie Stecker, Kabel etc. Zudem ist das Risiko einer elektromagnetischen Unverträglichkeit (EMV) minimiert, da über das Kabel nur Signale mit geringer Leistung und die Stromversorgung geführt werden.

Zwischen Elektronikteil und Verneblerteil dichtete zum Beispiel ein Dichtungsring die Verbindung ab. In manchen Ausführungsformen dichtet der Dichtungsring zugleich auch das Elektronikteil ab

Das Elektronikteil und das Verneblerteil sind beispielsweise über einen Rastmechanismus einfach und intuitiv verbindbar.

Die Fassung hält den Aerosolgenerator mit minimalen Einspannkräften in Position, dichtet das Medikamentenreservoir gegen den Flansch/das Anschlussstück und dichtet das Medikamentenreservoir gegen den Aerosolgenerator.

Die Erfindung betrifft auch ein Verfahren zur Steuerung der Aerosolerzeugung. Das Verfahren umfasst dabei zumindest einen der Schritte des Findens der Resonanzfrequenz des Aerosolgenerators, das Bestimmen des optimalen Wirkungsgrades und/oder das Einstellen der Pulsweite und/oder das Einstellen der Menge an erzeugtem/zu erzeugendem Aerosol.

Die Resonanzfrequenz des Aerosolgenerators wird bestimmt durch die Vorgabe verschiedener Anregungsfrequenzen und die gleichzeitige Bestimmung der Spannung am Aerosolgenerator bzw. dem Schwingkreis. Bei einer maximalen, gemessenen Spannung ist die Resonanzfrequenz erreicht. Die Resonanzfrequenz ist auch dadurch gekennzeichnet, dass die gemessene Spannung bei Überschreiten und/oder Unterschreiten der Resonanzfrequenz abnimmt.

Der optimale Wirkungsgrad des Aerosolgenerators wird durch Strommessungen an der Primärseite und der Sekundärseite des Transformators bestimmt. Durch Anpassung der Pulsweite kann der optimale Wirkungsgrad eingestellt werden. Der Wirkungsgrad ergibt sich aus dem Verhältnis der gemessenen/bestimmten Leistung auf der Primärseite zu der Leistung auf der Sekundärseite des Transformators. Beispielsweise kann die Leistung über die Spannung und die jeweilige Stromstärke an der Primär- und/oder Sekundärseite des Transformators ermittelt werden.

Die Menge an zu erzeugendem Aerosol wird durch eine Einstellung der Pulsbreite des HF-Signals erreicht. Durch eine höhere Pulsweite ergibt sich, in gewissen Grenzen, eine größere Menge an erzeugtem Aerosol. Eine geringere Pulsweite ergibt eine geringere Menge an erzeugtem Aerosol. Die Pulsweite der Impulse, welche an dem Transformator anliegen, steuern dabei den Energieeintrag in das Schwingsystem des Aerosolgenerators. Eine höhere Pulsweite entspricht dabei also einem höheren Energieeintrag.

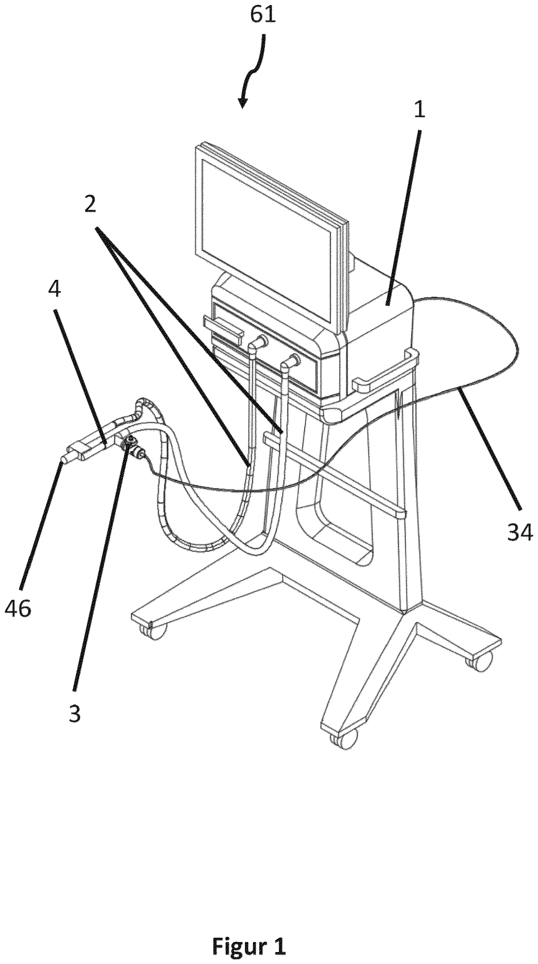

Die Erfindung wird anhand der Figuren 1 bis 11 beispielhaft näher beschrieben.

In Figur 1 ist ein Beatmungsgerät 1 dargestellt, welches in einem Beatmungssystem 61 mit dem Vernebler 3 verbunden ist. Das Beatmungsgerät 1 ist beispielsweise dazu eingerichtet, einen Atemgasstrom über einen Schlauch 2 zu einem Patienten zu fördern. Die Verbindung mit dem Patienten findet dabei zum Beispiel über ein Patienteninterface statt, welches beispielsweise über einen Maskenanschluss 46 mit dem Schlauchsystem und dem Beatmungsgerät 1 verbunden ist. Das beispielhaft gezeigte Beatmungsgerät 1 verfügt über ein Zwei-Schlauch-System, wobei durch einen Schlauch 2 das Atemgas zum Patienten gefördert wird und durch den anderen Schlauch das ausgeatmete Atemgas des Patienten abgeleitet wird. Neben einem solchen Zwei-Schlauch-System können auch andere Arten von Verbindung zwischen Patient und Beatmungsgerät 1 genutzt werden, beispielsweise mit einem Leckage-System, durch welches der Patient ausatmet.

Das Beatmungsgerät 1 verfügt über zumindest eine Benutzerschnittstelle, über welche zumindest Daten, Informationen und/oder Vorgaben eingegeben werden können, welche die Beatmung und gegebenenfalls die Erzeugung von Aerosol durch den Vernebler 3 betreffen.

Das Beatmungsgerät 1 ist beispielsweise dazu eingerichtet den Patienten bei der Atmung zu unterstützen und/oder auch die Atmung des Patienten vorzugeben und/oder eine künstliche Beatmung durchzuführen. Dazu weist das Beatmungsgerät 1 zumindest eine Atemgasquelle zum Bereitstellen von Atemgas auf, wobei optional auch zusätzliche Gasquellen, zum Beispiel für Sauerstoff, genutzt werden können oder in das Beatmungsgerät 1 integriert sind. Das Atemgas kann dabei druck- und/oder flussgesteuert zum Patienten gefördert werden, wobei das Beatmungsgerät 1 über entsprechende Steuer- und Sensoreinheiten verfügt. Darüber hinaus ist das Beatmungsgerät 1 dazu ausgebildet und eingerichtet Atemphasen, wie zum Beispiel Inspiration und Exspiration, zu erkennen und die Bereitstellung des Atemgases anhand der Atemphase zu steuern. Auch kann das Beatmungsgerät 1 dazu ausgebildet sein, verschiedene Atemwegsprobleme, wie zum Beispiel Blockaden der Atemwege, Apnoen, etc., zu erkennen und daraufhin die Atemgassteuerung entsprechend anpassen. Das Beatmungsgerät 1 verfügt beispielsweise auch über eine Steuereinheit, über welche Vorgaben zur Aerosolgeneration an die Steuerelektronik 13 weitergegeben werden.

Der erfinderische Vernebler 3 ist bevorzugt mit dem Schlauch 2 verbunden, durch welchen das Atemgas während der Inspiration zu dem Patienten gefördert wird. In einer vorteilhaften Weise ist der Vernebler 3 möglichst nah an dem Maskenanschluss 46 angeordnet. Beispielhaft ist der Vernebler 3 über ein T-Stück 4 mit dem Schlauch 2 zur Inspiration verbunden. Zur Energieversorgung und zur Übermittlung von Signalen, beispielsweise zur Steuerung des Verneblers 3, ist der Vernebler 3 über ein Kabel 34 mit dem Beatmungsgerät 1 verbunden. In manchen Ausführungsformen wird der Vernebler 3 über einen externen und/oder integrierten Akku/Batterie mit Energie versorgt.

Der Vernebler 3 ist als Schwingmembranvernebler ausgebildet. Dazu verfügt der Vernebler 3 über zumindest einen Aerosolgenerator 16 - zumindest bestehend aus einer Substratscheibe 7, einer perforierten Membran 5 sowie einem Piezoelement 6 - über welchen ein Aerosol durch Zerstäubung einer Flüssigkeit 9 erzeugt werden kann. Dazu wird die Membran 5 über das Piezoelement 6 in Schwingungen versetzt, wobei von einer Seite der Membran 5 eine Flüssigkeit 9 an die Membran 5 geführt wird, welche durch die Öffnungen 14 der Membran 5 gedrückt wird bzw. die Membran 5 wird gegen die Flüssigkeit 9 gedrückt, wodurch die Flüssigkeit 9 zum Teil durch die Öffnungen 14 gedrückt wird und in kleinen Aerosoltröpfchen 15 abreißt. Die Flüssigkeit 9 kann dabei jedwedes flüssiges Medium sein, inklusive gelöster Stoffe. Insbesondere sind Medikamente und/oder Wirkstoffe, sowie deren Lösungen, Mischungen und/oder Verdünnungen als Flüssigkeit 9 einsetzbar. In manchen Ausführungsformen sind auch Suspensionen und/oder Emulsionen als Flüssigkeit 9 nutzbar, wobei insbesondere bei Suspensionen die Partikelgröße die Größe der Öffnungen 14 nicht überschreiten sollte.

Als Material für die Membran 5 kann beispielsweise Edelstahl verwendet werden. Eine Edelstahlmembran wird beispielsweise in einem Laserprozess strukturiert. In manchen Ausführungsformen sind auch Nickel und/oder Nickel-Palladium-Legierungen und/oder andere Metalle/Metall-Legierungen als Membranmaterialien denkbar. Membranen aus Nickel und/oder Nickel-Palladium-Legierungen können beispielsweise über ein Elektroforming-Verfahren hergestellt werden. Auch andere Materialen, wie beispielsweise Kunststoffe/Polymere und/oder Silizium können grundsätzlich als Membranmaterialien eingesetzt werden.

Über das Kabel 34 kann das Beatmungsgerät 1 Signale zur Steuerung an den Vernebler 3 übermitteln. Beispielsweise kann so gesteuert werden, wann der Vernebler 3 ein Aerosol erzeugen soll. Auch die Dauer der Aerosolerzeugung und die Menge an erzeugtem Aerosol kann beispielsweise von dem Beatmungsgerät 1 vorgegeben werden. Dazu ist zum Beispiel die Steuerelektronik 13 des Verneblers 3 so eingerichtet, die Signale des Beatmungsgerätes 1 entsprechend zu interpretieren und umzusetzen. In manchen Ausführungsformen kann das Beatmungsgerät 1 auch Sensordaten, beispielsweise Druck- und/oder Flussdaten, an den Vernebler 3 weiterleiten, wobei die Steuerelektronik 13 des Verneblers 3 so eingerichtet ist, die Sensordaten zu interpretieren und selbstständig Zeitpunkt, Dauer und/oder Menge an Aerosolerzeugung zu bestimmen. In manchen Ausführungsformen kann die Steuerelektronik 13 auch zumindest teilweise im Beatmungsgerät 1 angeordnet sein. Beispielsweise werden dann lediglich elektrische Impulse an den Vernebler 3 weitergeleitet.

Beispielsweise ist das Beatmungsgerät 1 so eingerichtet, dass anhand des Atemzyklus der Vernebler 3 zur Aerosolerzeugung angesteuert wird. In manchen Fällen wird beispielsweise nur zur Inspiration Aerosol erzeugt. Dazu kann beispielsweise mit Beginn der Inspiration auch die Aerosolerzeugung gestartet werden. Auch ist denkbar, dass beispielsweise kurz vor Ende der Exspiration bereits mit der Aerosolerzeugung begonnen wird, sodass bereits mit Beginn der Inspiration eine gewisse Menge an Aerosol zur Verfügung steht. Auch kann beispielsweise die Aerosolerzeugung bereits vor Ende der Inspiration beendet werden, zum Beispiel, wenn der Atemfluss zum Ende der Inspiration abnimmt und kaum noch Aerosol eingeatmet werden würde. Auch eine Steuerung anhand erkannter Atmungsprobleme kann möglich sein.

Auch kann vorgesehen sein, dass das Beatmungsgerät 1 und/oder der Vernebler 3 die zu erzeugende Aerosolmenge anhand von weiteren medizinischen Daten und/oder Vorgaben steuert. In manchen Ausführungsformen kann beispielsweise eine zeitlich gesteuerte Aerosolerzeugung eingerichtet werden, beispielsweise wird zu bestimmten Zeitpunkten eine gewisse Menge an Aerosol erzeugt, beispielsweise als regelmäßige Medikamentengabe. In manchen Ausführungsformen wird beispielsweise das Beatmungsgerät 1 und/oder der Vernebler 3 mit weiteren medizinischen und/oder physiologischen Daten - z.B. Herzfrequenz, Sauerstoffsättigung, Schlaf-/Wachphasen, Körpertemperatur, etc. - versorgt, worauf eine Anpassung und/oder Aktivierung der Aerosolerzeugung erfolgt.

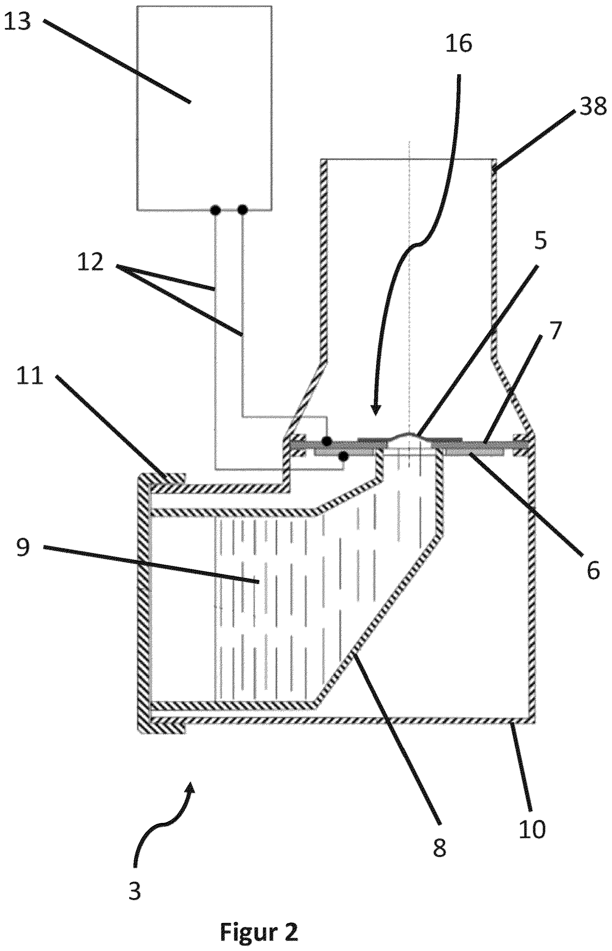

Figur 2 zeigt schematisch den Aufbau einer beispielhaften Ausführungsform des erfinderischen Verneblers 3. In einem Gehäuse 10 ist dabei der Aerosolgenerator 16 angeordnet, wobei der Aerosolgenerator 16 aus einer Substratscheibe 7, einer perforierten Membran 5 und einem Piezoelement 6 besteht. Beispielsweise ist die Substratscheibe 7 im Wesentlichen kreisrund und weist in der Mitte einen Ausschnitt bzw. eine Öffnung auf. Diese Öffnung wird von der perforierten Membran 5 verschlossen, wobei die Membran 5 mit der Substratscheibe 7 verbunden wird. Die Verbindung zwischen Substratscheibe 7 und Membran 5 kann beispielsweise durch Verkleben, Einpressen, Aufpressen, Schweißen oder andere geeignete Methoden erfolgen. Die perforierte Membran 5 ist beispielhaft gewölbt und so auf die Substratscheibe 7 gesetzt, dass die Flüssigkeit 9 von hinten in die Wölbung an die Membran herangeführt wird. Aus Sicht der Flüssigkeit 9 ist die Membran 5 also nach außen gewölbt. Ebenfalls auf der Substratscheibe 7 ist das Piezoelement 6 angeordnet. Beispielsweise ist das Piezoelement 6 ebenfalls kreisrund und weist eine Öffnung in der Mitte auf. Beispielsweise ist das Piezoelement 6 so ausgeführt, dass der Durchmesser der Öffnung des Piezoelements 6 größer ist als der Durchmesser der Öffnung der Substratscheibe 7 und der Durchmesser der Substratscheibe 7 ist größer als der Durchmesser des Piezoelements 6. Die Substratscheibe 7 ragt also über den äußeren Rand des Piezoelements 6 hinaus und ragt auch über den inneren Rand (also den Rand der Öffnung) des Piezoelements 6 hinaus. Die Substratscheibe 7 steht also über beide Ränder des Piezoelements 6 über. Die Substratscheibe 7 und das Piezoelement 6 sind miteinander verbunden, beispielsweise durch Verkleben, Aufpressen, Einpressen, Verschweißen oder andere geeignete Verbindungsmethoden.

Durch das Anlegen einer Spannung verformt sich das Piezoelement 6 und induziert dadurch auch eine Deformation der verbundenen Substratscheibe 7. Beispielsweise kann sich das Piezoelement 6 bei angelegter Spannung zusammenziehen oder ausdehnen, wodurch die Substratscheibe 7 verbogen wird und die Membran 5 aus der Ruhelage ausgelenkt und gegebenenfalls ebenfalls etwas gedehnt oder gestaucht wird.

In dem Gehäuse 10 ist weiter ein Flüssigkeitsreservoir 8 angeordnet zur Vorhaltung der Flüssigkeit 9. Das Flüssigkeitsreservoir 8 ist so eingerichtet, dass die Flüssigkeit 9 auf einer Seite an die Membran 5 des Aerosolgenerators 16 herangeführt wird. Der Aerosolgenerator 16 stellt dabei eine Abgrenzung zwischen dem Flüssigkeitsreservoir 8 und dem Atemgas dar, welche durch den Schlauch 2 bzw. das T-Stück 4 geleitet wird (siehe Figur 1). Das Flüssigkeitsreservoir 8 wird beispielhaft mit einem Deckel 11 verschlossen. In manchen Ausführungsformen kann der Deckel 11 auch

Über ein Anschlussstück 38 wird der Vernebler 3 mit dem Schlauchsystem bzw. dem T-Stück 4 verbunden und das erzeugte Aerosol in das Atemgas und beispielsweise zu einem Patienten geleitet.

Die Steuerelektronik 13 des Verneblers 3 ist beispielhaft über elektrische Verbindungen 12 mit dem Aerosolgenerator 16 verbunden. Die Steuerelektronik 13 steuert zum Beispiel die Aerosolgenerationsrate und -menge. Auch ist die Steuerelektronik 13 mindestens dazu eingerichtet eine optimale Anregungsfrequenz des Aerosolgenerators zu bestimmen und einzustellen. Als optimale Anregungsfrequenz kann beispielsweise die Resonanzfrequenz des Aerosolgenerators 16, optional in Verbindung mit weiteren Elementen, angesehen werden. Zum Beispiel kann auch die Resonanzfrequenz des Schwingkreises 44 als optimale Anregungsfrequenz angesehen werden. Auch kann über die Steuerelektronik 13 die Amplitude der Deformation des Piezoelements 6 gesteuert werden, wodurch die Aerosolgeneration direkt beeinflusst wird.

In manchen Ausführungsformen kann der Vernebler 3 auch mehrere, unabhängig voneinander steuerbare Aerosolgeneratoren 16 umfassen. Beispielsweise können diese verschieden perforierte Membranen aufweisen und/oder von verschiedenen Flüssigkeitsreservoirs 8 bedient werden. Beispielsweise können so auch mehrere verschiedene Wirkstoffe unabhängig voneinander dem Atemgas als Aerosol beigemischt werden und je nach Bedarf dosiert werden.

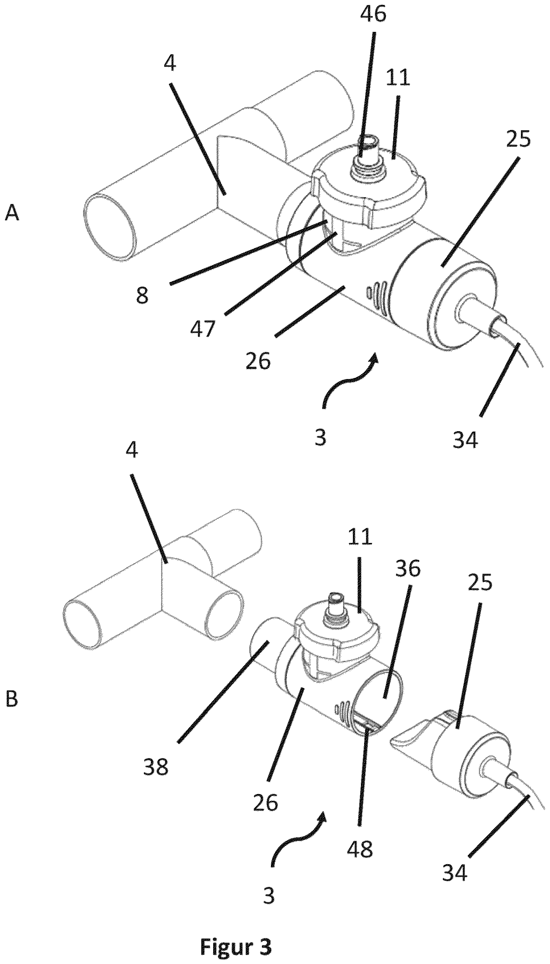

Die Figur 3 zeigt eine beispielhafte Ausführungsform des Verneblers 3 und des T-Stückes 4 in einer zusammengesetzten Perspektivansicht (Figur 3 A) und einer Explosionsansicht (Figur 3 B). Der Vernebler 3 besteht dabei aus zwei Hauptbestandteilen, dem Verneblerteil 26 und dem Elektronikteil 25. Der Elektronikteil 25 beinhaltet beispielsweise die Steuerelektronik 13 des Verneblers 3 und kann wiederverwendet werden. Der Verneblerteil 26 umfasst mindestens den Aerosolgenerator 16 sowie das Flüssigkeitsreservoir 8. Der Verneblerteil 26 kann beispielsweise als Einweg-Produkt geplant sein, sodass das Verneblerteil 26 nur für einen Patienten verwendet wird und bei Patientenwechsel ein neues Verneblerteil 26 eingesetzt wird, wobei das Elektronikteil 25 auch für mehrere Patienten genutzt werden kann. In manchen Ausführungsformen kann der Verneblerteil 26 ebenfalls für eine Mehrfachverwendung ausgebildet sein, beispielsweise durch Verwendung von leicht zu reinigenden Materialien und einer einfachen Demontage. In manchen Ausführungsformen kann der Verneblerteil 26 zumindest teilweise wiederverwendet werden, so kann beispielsweise der Aerosolgenerator 16 austauschbar sein, während das Gehäuse 10 inkl. Anschlussstück 38 wiederverwendet werden.

In der gezeigten Ausführungsform ist am Flüssigkeitsreservoir 8 beispielhaft eine Füllstandanzeige 47 angeordnet. Die Füllstandanzeige 47 kann zum Beispiel durch eine zumindest teilweise transparente Wand des Flüssigkeitsreservoirs 8 realisiert werden, durch welche der Pegelstand der Flüssigkeit 9 gesehen werden kann. In manchen Ausführungsformen sind dazu auch zusätzliche Markierungen angeordnet, beispielsweise um einen maximalen und/oder minimalen Füllstand zu markieren.

Verschlossen wird das Flüssigkeitsreservoir 8 mit einem Deckel 11. Der Deckel 11 kann entfernt werden um das Flüssigkeitsreservoir 8 mit einer Flüssigkeit 9 zu befüllen und/oder nachzufüllen. Am Deckel 11 kann optional auch eine Fülleinrichtung 46 angeordnet sein, über welche zum Beispiel während des Betriebs Flüssigkeit 9 nachgefüllt werden kann. Auch eine Befüllung über die Fülleinrichtung 46 ist möglich. Die Fülleinrichtung 46 kann beispielsweise ein Stutzen sein, auf den eine Spritze aufgesteckt werden kann. In manchen Ausführungsformen ist die Fülleinrichtung 46 auch eine verschließbare Öffnung und/oder ein Septum, durch welches mit einer Spritzennadel durchstochen wird und sich in der Regel nach entfernen der Nadel wieder selbst verschließt. In manchen Ausführungsformen sind für den Deckel 11 (inkl. Fülleinrichtung 46) und/oder das Flüssigkeitsreservoir 8 Einrichtungen für einen Druckausgleich vorgesehen, sodass bei einer Befüllung über beispielsweise eine Spritze kein Überdruck entsteht und ungewollt Flüssigkeit 9 durch die Membran 5 gedrückt wird.

Über das Anschlussstück 38 am Verneblerteil 26 wird der Vernebler 3 beispielsweise mit dem T-Stück 4 verbunden, welches gasleitend mit dem Schlauch 2 verbunden ist. Über das T-Stück 4 wird das generierte Aerosol in den Atemgasstrom eingeleitet und beispielsweise zu einem Patienten gefördert/geleitet. Bevorzugt sind das Anschlussstück 38 und das T-Stück 4 so ausgebildet, dass die Aerosolerzeugung möglichst nah zum Atemgasstrom erfolgt, sodass das Aerosol nur eine geringe Strecke zurücklegt bis es in den Atemgasstrom übergehen kann.

Das Verneblerteil 26 weist zudem eine Einführöffnung 36 auf, in welche das Elektronikteil 25 eingesteckt und mit dem Verneblerteil 26 verbunden wird. Beispielsweise ist dafür im Inneren des Verneblerteils 26 eine Führungsschiene 48 angeordnet, welche unter anderem zur korrekten Einführung des Elektronikteils 25 über ein der Führungsschiene 48 komplementären Führungselement 49. In manchen Ausführungsformen sind in der Einführöffnung 36 auch weitere Führungsschienen 48 und/oder Führungselemente 49 angeordnet, welche jeweils komplementär zu den entsprechenden Elementen an dem Elektronikteil 25 ausgebildet sind.

Das Elektronikteil 25 wird beispielsweise in die Einführöffnung 36 des Verneblerteils 26 geführt und kann dort beispielsweise dort einrasten. Das Elektronikteil 25 umfasst zumindest die Steuerelektronik 13 des Verneblers 3. Über elektrische Leitungen 12 wird ein Kontakt zwischen dem Aerosolgenerator 16 im Verneblerteil 26 und der Steuerelektronik 13 im Elektronikteil 25 hergestellt. Über ein Kabel 34 kann das Elektronikteil 25 beispielsweise mit einem Beatmungsgerät 1 verbunden werden. Über das Beatmungsgerät 1 kann so zum Beispiel eine Steuerung, beispielsweise im Sinne von Befehlen betreffend den Zeitpunkt, die Dauer und die Menge der Aerosolerzeugung, des Verneblers 3 erfolgen und/oder die Energieversorgung des Verneblers 3. Es können also Vorgaben zur Aerosolerzeugung durch das Beatmungsgerät (1) eingegeben werden, welche vom Vernebler 3 bzw. der Steuerelektronik 13 empfangen und umgesetzt werden.

In manchen Ausführungsformen kann der Vernebler 3 unabhängig von dem Beatmungsgerät 1 betrieben werden. Dazu verfügt der Vernebler 3 beispielsweise eine eigene Energieversorgung in Form eines Akkus/einer Batterie und/oder einem Anschluss für das lokale Stromnetz. Eine Aktivierung/Deaktivierung des Verneblers 3 erfolgt dann über die Verbindung mit einer Energieversorgung und/oder durch einen Schalter. Es ist zudem denkbar, dass der Vernebler 3 über Bedienelemente verfügt, über welche verschiedene Optionen wie z.B. zu erzeugende Aerosolmenge eingestellt werden können. In manchen Ausführungsformen ist auch eine Kommunikation des Verneblers 3 mit dem Beatmungsgerät 1 über Funk möglich.

Die in Figur 3 gezeigte Ausführungsform des Verneblers 3 ist zweiteilig, bestehend aus Elektronikteil 25 und Verneblerteil 26, welche voneinander getrennt werden können. In manchen Ausführungsformen ist der Vernebler 3 auch einteilig ausgeführt. Damit der Vernebler 3 wiederverwendet werden kann, wäre dabei zumindest an einen tauschbaren und/oder entnehmbaren Aerosolgenerator 16 gedacht.

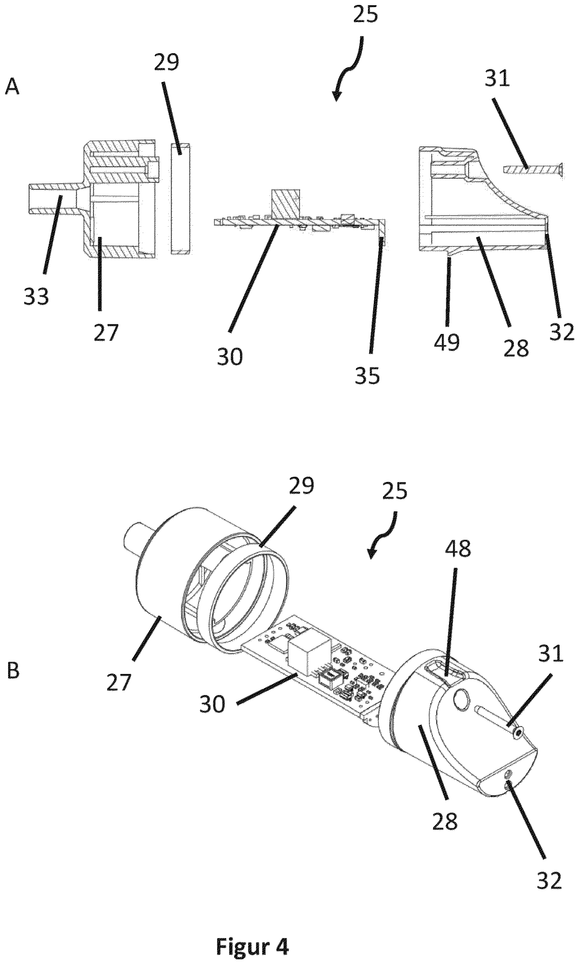

Eine beispielhafte Ausführungsform des Elektronikteils 25 ist in Figur 4 in einer Explosionsansicht dargestellt. Figur 4 A zeigt einen Schnitt durch das Elektronikteil 25 und die Figur 4 B eine perspektivische Ansicht.

Das Elektronikteil 25 ist beispielhaft aus zwei Gehäuseteilen 27, 28 zusammengesetzt, in welchen unter anderem die Platine 30 mit der Steuerelektronik 13 angeordnet ist. Die beiden Gehäuseteile 27, 28 werden unter anderem durch eine Schraube 31 miteinander fixiert. Alternativ oder ergänzend kann auch eine Fixierung der Gehäuseteile 27, 28 über eine Steck- und/oder Klemm- und/der Rastverbindung realisiert werden. In der beispielhaft gezeigten Ausführungsform wird das Gehäuseteil 28 zumindest teilweise in das Gehäuseteil 27 gesteckt. Zur zusätzlichen Abdichtung, beispielsweise gegen Feuchtigkeit und/oder Flüssigkeiten wird ein Dichtungsring 29 zwischen den Gehäuseteilen 27, 28 angeordnet.

Das Gehäuseteil 27 umfasst einen Kabelanschluss 33 worüber das Kabel 34 mit der Platine 30 verbunden werden kann. Beispielsweise kann der Kabelanschluss 33 als Durchführung ausgebildet sein, sodass das Kabel 34 zumindest teilweise durch die Durchführung geschoben werden kann. Das Kabel 34 und/oder der Kabelanschluss 33 sind gegebenenfalls so ausgebildet, dass der Kabelanschluss 34 gegen Eindringen von Feuchtigkeit und/oder Flüssigkeit in das Elektronikteil 25 dicht ist. Beispielsweise läuft der Kabelanschluss 34, als Durchführung ausgeführt, leicht konisch zusammen, sodass das Kabel 33 beim Durchführen dicht eingeklemmt wird. In manchen Ausführungsformen enthält das Gehäuseteil 27 Elemente, an denen die Platine 30 befestigt werden kann. Beispielsweise kann die Platine 30 über Schraubverbindungen in dem Gehäuseteil 27 fixiert werden. In manchen Ausführungsformen sind alternativ oder ergänzend Steckelemente für die Platine 30 vorgesehen. In manchen Ausführungsformen verfügt die Platine 30 über Steckverbindungen für das Kabel 34. So kann das Kabel 34 beispielsweise lösbar mit dem Elektronikteil 25 verbunden sein. In manchen Ausführungsformen kann hingegen vorgesehen sein, dass das Kabel 34 fest mit dem Elektronikteil 25, also mit Platine 30 und/oder Gehäuseteil 27, verbunden ist.

Die Platine 30 umfasst zumindest die Steuerelektronik 13 des Verneblers 3. Zusätzlich sind, im Falle einer Verbindung über ein Kabel 34, zumindest weitere Elemente vorgesehen, mit denen die Leitungen des Kabels 34 mit der Platine 30 verbunden werden. Weiter ist an der Platine 30 zumindest ein Kontaktelement 35 angeordnet, über welches der Kontakt zwischen der Platine 30 und dem Aerosolgenerator 16 hergestellt wird. Das Kontaktelement 35 umfasst zusätzlich zumindest ein Dichtelement, beispielsweise eine Gummidichtung.

Der Kontakt mit dem Aerosolgenerator 16 erfolgt beispielsweise durch Öffnungen 32, welche im Gehäuseteil 28 ausgebildet sind. Die elektrischen Verbindungen 12 werden beispielsweise durch die Öffnungen 32 mit dem Kontaktelement 35 verbunden. Im Bereich der Öffnungen 32 wird das Innere des Elektronikteils 25 über die Dichtelemente am Kontaktelement 35 gegenüber Feuchtigkeit und/oder eindringende Flüssigkeit abgedichtet. In manchen Ausführungsformen sind alternativ oder ergänzend an und/oder in den Öffnungen 32 Dichtelemente, wie z.B. Gummidichtungen, angeordnet um gegen eindringende Feuchtigkeit und/oder Flüssigkeit abzudichten. Beispielsweise weisen die Öffnungen 32 eine Gummidurchführung auf, welche rund um die elektrischen Verbindungen 12 abdichten.

Optional sind auch in dem Gehäuseteil 28 Vorrichtungen angeordnet um die Platine 30 zu haltern. Beispielsweise können dazu Schraubpunkte und/oder Steckelemente vorgesehen sein. In manchen Ausführungsformen ist der Bereich des Gehäuseteils 28, in welchem das Kontaktelement 35 der Platine 30 angeordnet wird, so ausgebildet, dass das Kontaktelement 35 leicht verklemmt wird.

Das Gehäuseteil 28 verfügt beispielhaft über eine Führungsschiene 48 sowie ein Führungselement 49. Die Führungsschiene 48 ist beispielhaft so ausgebildet, dass sie komplementär zu einem Führungselement in der Einführöffnung 36 des Verneblerteils 26 ist. Das Führungselement 49 ist so ausgebildet, dass es zum einen in der Führungsschiene 48 des Verneblerteils 26 geführt wird, zum anderen kann das Führungselement 49 auch in zumindest einer Position in der Führungsschiene 48 des Verneblerteils 26 einrasten. So kann zum Beispiel ein Feedback entstehen, dass Verneblerteil 26 und Elektronikteil 25 korrekt zusammengesteckt sind.

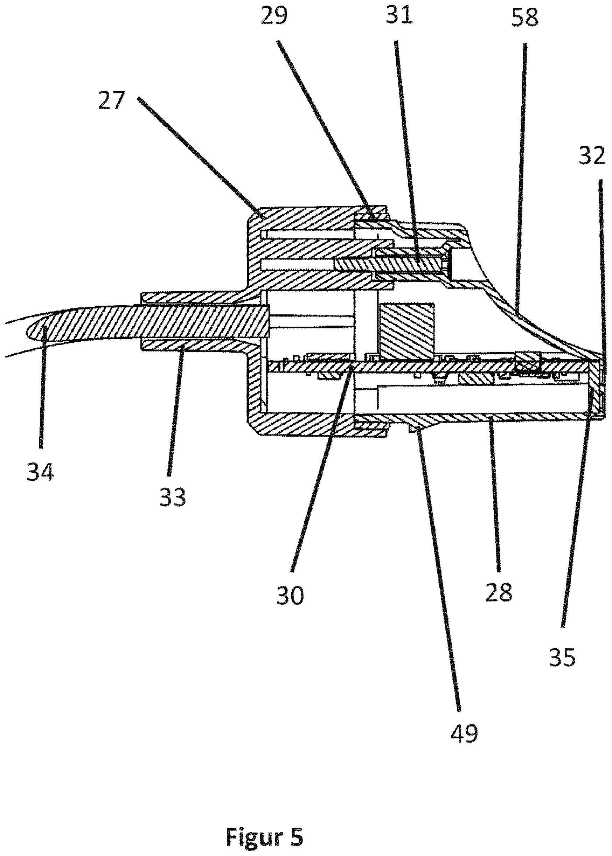

Eine zusammengesetzte Form des Elektronikteils 26 ist bespielhaft in einem Schnitt in Figur 5 dargestellt. Das Gehäuseteil 27 ist über die Schraube 31 mit dem Gehäuseteil 28 fixiert. In dem Bereich, in welchem das Gehäuseteil 28 teilweise in das Gehäuseteil 27 gesteckt ist, ist zwischen den Gehäuseteilen 27, 28 ein Dichtungsring 29 angeordnet.

Die Platine 30 ist so angeordnet, dass das Kontaktelement 35 im Bereich der Öffnungen 32 des Gehäuseteils 28 angeordnet ist. Konkret ist das Kontaktelement 35 so platziert, dass durch die Öffnungen 32 eine elektrische Verbindung 12 zu dem Aerosolgenerator 16 des Verneblerteils 26 hergestellt werden kann. Im Bereich des Kontaktelements 35 und/oder in und/oder an den Öffnungen 32 sind Dichtelemente angeordnet, welche gegen ein Eindringen von Feuchtigkeit und/oder Flüssigkeit durch die Öffnungen 32 in den Innenraum des Elektronikteils 25 abdichten.

Durch den Kabelanschluss 33 ist das Kabel 34 mit dem Elektronikteil 25 verbunden. Das Kabel 34 steckt dabei so in der Durchführung des Kabelanschlusses 33, dass hier gegen eindringende Flüssigkeit und/oder Feuchtigkeit abgedichtet ist.

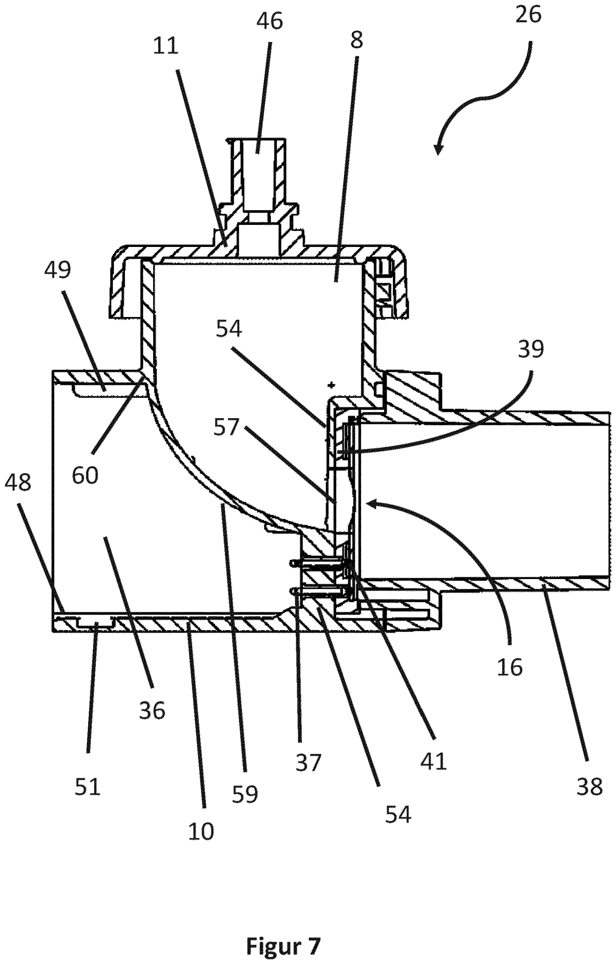

Das Gehäuseteil 28 weist beispielsweise einen Bogenabschnitt 58 auf, welcher dadurch gekennzeichnet ist, dass die Gehäusewand in diesem Bereich gebogen verläuft. In manchen Ausführungsformen ist dieser Bogenabschnitt 58 auf die Wand 59 des Verneblerteils 26 angepasst (siehe Figur 7), kann also statt bogenförmig auch schräg und/oder zumindest abschnittsweise gerade verlaufen. Sind Bogenabschnitt 58 und Wand 59 aufeinander abgestimmt und verlaufen bogenförmig, so weisen beide zum Beispiel korrespondierende Radius auf.

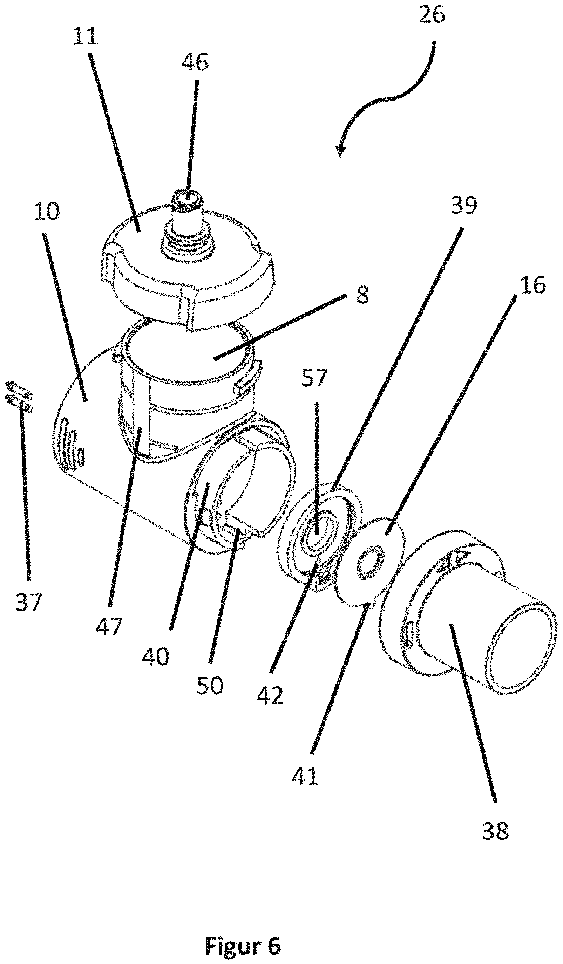

Eine beispielhafte Ausführungsform des Verneblerteils 26 in einer perspektivischen Explosionsansicht ist in Figur 6 dargestellt. Das Verneblerteil 26 umfasst unter anderem beispielhaft ein Gehäuse 10 mit dem Flüssigkeitsreservoir 8, welches über einen Deckel 11 verschlossen werden kann. Auch umfasst das Verneblerteil 26 den Aerosolgenerator 16, welcher in einer Fassung 39 im Gehäuse 10 gelagert wird sowie ein Anschlussstück 38, welches über eine Anschlussaufnahme 40 mit dem Gehäuse 10 verbunden wird und die Fassung 39 mit dem Aerosolgenerator 16 im Gehäuse 10 fixiert. Die Fassung 39 mit Aerosolgenerator 16 wird beispielsweise bis zu der Begrenzung 54 (siehe Figur 7) geschoben. Dazu ist beispielhaft eine Führungsnut 50 im Gehäuse 10 des Verneblerteils 26 ausgebildet. Das Anschlussstück 38, beispielsweise als Flansch ausgebildet, wird darüber gesetzt bzw. auf die Anschlussaufnahme 40 gesteckt und beispielsweise über einen Bajonettverschluss und/oder eine einfache Steckverbindung fixiert. So können am Anschlussstück 38 beispielsweise über Rastmulden und/oder andere Rastelemente angeordnet sein, welche an komplementären Rastelementen der Anschlussaufnahme 40 einrasten. Das Anschlussstück 38, die Fassung 39 mit Aerosolgenerator 16 sowie die Begrenzung 56 und die Anschlussaufnahme 40 des Gehäuses 10 sind dabei so dimensioniert, dass die Fassung 39 mit Aerosolgenerator 16 von dem Anschlussstück 38 fixiert werden.

Die Fassung 39 für den Aerosolgenerator 16 weist zumindest eine Öffnung 57 auf, über welche die Flüssigkeit 9 aus dem Flüssigkeitsreservoir 8 an die perforierte Membran 5 des Aerosolgenerators 16 herangeleitet wird. Zudem weist die Fassung zumindest eine Öffnung 42 auf, durch welche die Kontaktstifte 37 gesteckt werden können um eine elektrische Verbindung 12 zwischen dem Aerosolgenerator 16 (über den Elektronikkontakt 41) und dem Kontaktelement 35 der Platine 30 herzustellen. Entsprechend komplementäre Öffnungen sind ebenfalls in der Begrenzung 54 angeordnet.

Der Deckel 11 verfügt beispielhaft über eine Fülleinrichtung 46 über welche zum Beispiel während des Betriebs Flüssigkeit 9 in das Flüssigkeitsreservoir (nach)gefüllt werden kann. Beispielhaft verfügt das Flüssigkeitsreservoir 8 über eine Füllstandanzeige 47, über welche bei Bedarf der Füllstand an Flüssigkeit 9 kontrolliert werden kann. Der Deckel 11 wird in der gezeigten Ausführungsform beispielhaft über einen Bajonettverschluss am Gehäuse 10 lösbar fixiert. In manchen Ausführungsformen sind alternativ oder ergänzend auch andere Verschlussmöglichkeiten für den Deckel 11 denkbar.

Ein Schnitt durch eine beispielhafte Ausführungsform des Verneblerteils 26 ist in Figur 7 dargestellt. Über eine Wand 59 ist das Flüssigkeitsreservoir 8 von der Einführöffnung 36 für das Elektronikteil 25 getrennt. Innerhalb der Begrenzung 54 sind Durchführungen ausgebildet, sodass über die Kontaktstifte 37 eine elektrische Verbindung 12 zwischen dem Elektronikkontakt 41 des Aerosolgenerators 16 und dem Kontaktelement 35 der Platine 30 hergestellt werden kann. Der Aerosolgenerator 16 ist in der Fassung 39 im Gehäuse 10 des Verneblerteils 26 gelagert. Gehalten wird die Fassung 39 mitsamt Aerosolgenerator 16 durch das Anschlussstück 38, welches die Fassung 39 gegen die Begrenzung 54 drückt. Die Fassung 39 und/oder die Begrenzung 54 weisen optional Dichtelemente auf, sodass keine Flüssigkeit 9 aus dem Flüssigkeitsreservoir 8 ungewollt in das Anschlussstück 38 gelangt. In manchen Ausführungsformen ist zwischen der Fassung 39 und der Begrenzung 54 und/oder zwischen der Fassung 39 bzw. dem Aerosolgenerator 16 und dem Anschlussstück 38 ein zusätzlicher Dichtungsring angeordnet. In manchen Ausführungsformen ist insbesondere der Elektronikkontakt 41 und/oder die Durchführung 42 so ausgebildet, dass keine Feuchtigkeit und/oder Flüssigkeit in die Einführöffnung 36 bzw. zu dem Elektronikteil 25 gelangt. In manchen Ausführungsformen ist dazu insbesondere der Bereich des Elektronikkontaktes 41 des Aerosolgenerators 16 entsprechend abgedichtet.

Im der Einführöffnung 36 ist zumindest eine Führungsschiene 48 angeordnet, welche komplementär zu dem Führungselement 49 des Elektronikteils 25 ausgebildet ist. In der Führungsschiene 48 ist zu dem mindestens eine Rastposition 51 ausgebildet, in welcher das Führungselement 49 des Elektronikteils 25 einrasten kann. Zusätzlich ist passend zu der Führungsschiene 48 des Elektronikteils 48 auch ein Führungselement 49 innerhalb der Einführöffnung 36 des Verneblerteils 26 ausgebildet.

Die Wand 59 zwischen Einführungsöffnung 36 und Flüssigkeitsreservoir 8 ist beispielhaft gebogen ausgeführt. Der Verlauf der Wand 59 entspricht dabei beispielsweise dem Bogenabschnitt 58 des Gehäuseteils 28 des Elektronikteils 25. Unter anderem kann so eine vereinfachte, richtig orientierte Einführung des Elektronikteils 25 in die Einführöffnung 36 des Verneblerteils 26 erreicht werden.

Die Wand 59 verläuft dabei auch so, dass sie mit dem Rand der Öffnung 57 oberhalb der Durchführung 42 für die Kontaktstifte 37 abschließt. Zum Beispiel bilden der Rand der Öffnung 57 und die Wand 59 eine Oberfläche. Die gebogene Form der Wand 59 in Kombination mit dem Abschluss auf Höhe des Randes der Öffnung 57 ermöglicht, dass zum einen ein geringes Totvolumen an Flüssigkeit entsteht, welche nicht zu einem Aerosol zerstäubt werden kann, da sie die Membran 5 des Aerosolgenerators 16 nicht erreicht. Zum anderen wird so auch vermieden, dass sich Flüssigkeitsreste in etwaigen Ecken ablagern können und ebenfalls nicht verbraucht werden können. In manchen Ausführungsformen ist die Wand 59 nicht gebogen, sondern verläuft gerad von dem Eckpunkt 60 zu der Öffnung 57. Die Wand 59 ist insbesondere so ausgebildet, dass die Flüssigkeit 9 im Flüssigkeitsreservoir 8 an den Aerosolgenerator 16 bzw. die Membran 5 herangeführt wird.

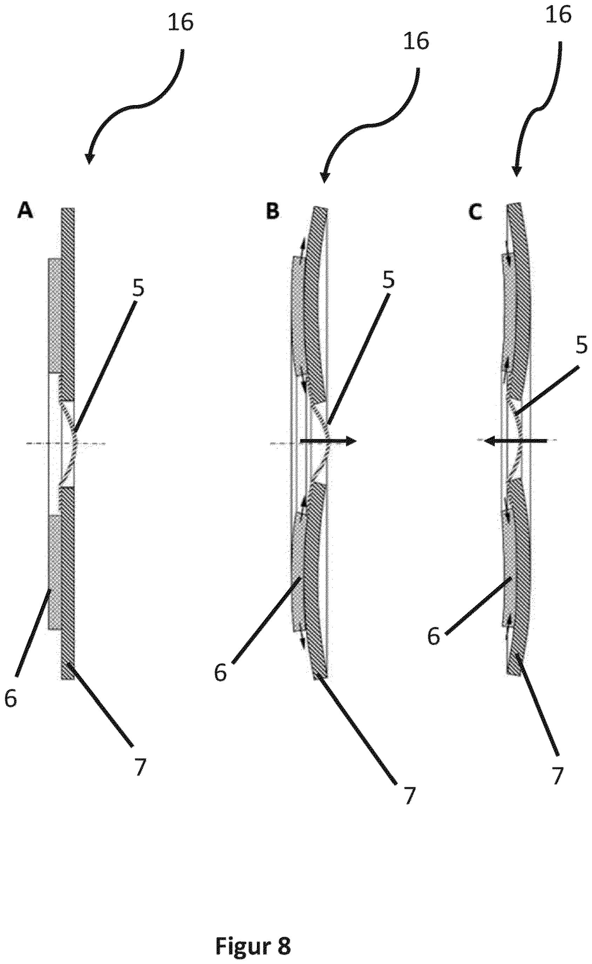

In Figur 8 ist eine beispielhafte Ausführungsform des Aerosolgenerators 16 im Ruhezustand (Figur 6 A) sowie in Betriebszuständen (Figuren 6 B und C) schematisch in einem Schnitt dargestellt.

Im Ruhezustand, wenn also keine Spannung anliegt bzw. auch, wenn eine angelegte Wechselspannung den Nullpunkt durchläuft, befindet sich das Piezoelement 6 in einem nicht-deformierten Zustand. Beispielhaft ist sind das Piezoelement 6 und die Substratscheibe 7 so ausgebildet, dass diese in einem nicht-deformierten Zustand jeweils eine Ebene bilden. Insbesondere sind sowohl Piezoelement 6 als auch Substratscheibe 7 als Scheibe ausgebildet und weisen jeweils eine zentrale Öffnung auf. Das Piezoelement 6 und die Substratscheibe 7 sind so miteinander verbunden, dass diese sich nicht unabhängig voneinander bewegen können. Das Piezoelement 6 ist beispielhaft fest auf einer Seite mit der Substratscheibe 7 verbunden. In einigen Ausführungsformen sind das Piezoelement 6 und die Substratscheibe 7 kreisförmig ausgebildet, wobei die Öffnung jeweils zentral ausgebildet ist. Die Substratscheibe 7 und das Piezoelement sind beispielsweise zentral übereinander angeordnet, sodass die Flächenmittelpunkte übereinanderliegen. Beispielhaft ist das Piezoelement 6 als Piezoaktor ausgeführt.

Mit der Substratscheibe 7 ist zudem auch die perforierte Membran 5 fest verbunden. In der beispielhaft gezeigten Ausführungsform ist die Membran 5 leicht gewölbt. Die Membran 5 ist so angeordnet, dass diese die Öffnung in der Substratscheibe 7 und/oder dem Piezoelement 6 verschließt. In manchen Ausführungsformen ist die Membran 5 so angeordnet, dass die Wölbung in die Öffnung der Substratscheibe 7 hineinragt und/oder durchragt. In der beispielhaft dargestellten Ausführungsform ist die Membran 5 mit der Substratscheibe 7 auf der gleichen Seite verbunden, auf welcher auch das Piezoelement 6 mit der Substratscheibe verbunden ist. Ist die Membran 5 gewölbt ausgeführt, so ist die Membran 5 in der Regel so angeordnet, dass die Wölbung von der Flüssigkeit 9 in dem Flüssigkeitsreservoir 8 weg zeigt. Die Flüssigkeit 9 kann also in die Wölbung fließen. In manchen Ausführungsformen kann die Membran 5 auch auf der Seite der Substratscheibe 7 angeordnet sein, welche der Seite mit dem Piezoelement 6 gegenüberliegt. In dem Fall würde die Wölbung der Membran 5 nicht in die Öffnung der Substratscheibe 7 hineinragen.

Die Membran 5 ist bevorzugt perforiert, weist also zumindest eine kleine Öffnung 14 auf, durch welche Flüssigkeit 9 zumindest zeitweise hindurch gedrückt werden kann um ein Aerosol zur erzeugen. Dabei reißen die Tropfen auf der Seite der Membran 5, welche der Flüssigkeit 9 abgewandt ist, ab. Bevorzugt weist die Membran 5 eine Mehrzahl an Öffnungen 14 in Form einer Perforation auf. Die Öffnungen 14 in der Membran 5 sind bevorzugt kreisförmig, können aber auch durchaus davon abweichen. Die Öffnungen 14 haben beispielsweise einen Durchmesser zwischen 0,1 µm und 300 µm (0,3 mm), wobei der Durchmesser je nach gewünschter durchschnittlicher Tropfengröße des Aerosols gewählt werden kann. Je größer die gewünschte Tropfengröße des Aerosols sein soll, desto größer können beispielsweise auch die Öffnungen 14 gewählt werden. An dieser Stelle sei darauf hingewiesen, dass auch weitere Faktoren wie zum Beispiel die Viskosität der Flüssigkeit 9 einen Einfluss auf die Tropfengröße haben können.

In manchen Ausführungsformen laufen die Öffnungen 14 in der Membran 5 konisch zu. Beispielhaft weisen die Öffnungen 14 der Membran 5 auf der Seite, an welcher die Flüssigkeit 9 herangeführt wird einen größeren Durchmesser auf als auf der Seite, an welcher die Flüssigkeit 9 von der Membran 5 als Aerosoltropfen abreißt.

Insbesondere sind die Öffnungen 14 so dimensioniert, dass im Ruhezustand, also zu Zeiten, wenn kein Aerosol erzeugt werden soll, im Wesentlichen keine Flüssigkeit 9 aus dem Flüssigkeitsreservoir 8 durch die Membran 5 tritt. Dabei ist anzumerken, dass während des Betriebs, also während Aerosol erzeugt werden soll, die Deformation des Aerosolgenerators 16 unter anderem den in Figur 8 A dargestellten Zustand durchläuft und anders als im Ruhezustand durchaus auch Flüssigkeit 9, beispielsweise in Form von kleinsten Tropfen bzw. als Aerosoltropfen, durch die Membran 5 treten kann.

Wird eine Spannung an das Piezoelement 6 angelegt, so verformt sich dieses, wie in Figur 8 B und C angedeutet. Dabei liegen zwischen den gezeigten Zuständen in Figur 8 B und C entgegengesetzte Spannungen an (z.B. in B eine positive und in C eine negative Spannung, oder umgekehrt).

In der in Figur 8 B gezeigten Darstellung wird durch das Anlegen einer Spannung an das Piezoelement 6 eine Deformation bzw. Verformung des Piezoelements 6 induziert. Das Piezoelement 6 dehnt sich dabei lateral aus, wie durch die Pfeile am Piezoelement 6 in Figur 8 B angedeutet. Dadurch, dass das Piezoelement 6 fest mit der Substratscheibe 7 verbunden ist, wird die in das Piezoelement 6 induzierte Deformation auf die Substratscheibe 7 übertragen, was zu einer Verbiegung der Substratscheibe 7 führt. Durch die Verbiegung der Substratscheibe 7 wird gleichzeitig auch die Membran 5 ausgelenkt, wie durch den Pfeil an der Membran 5 in Figur 8 B verdeutlicht. Dabei folgt die an der Membran 5 anstehende Flüssigkeit 9.

Wird die Spannung an dem Piezoelement 6 wieder verringert zieht sich das Piezoelement 6 wieder zusammen bzw. die Ausdehnung nimmt ab, wobei Substratscheibe 7 und Membran 5 der Bewegung entsprechend folgen. Bei Anlegen einer entgegensetzten Spannung an das Piezoelement 6 zieht sich das Piezoelement 6 zusammen, wie durch die Pfeile an dem Piezoelement 6 in Figur 8 C verdeutlicht. Die Substratscheibe 7 wird dadurch ebenfalls deformiert und lenkt gleichzeitig die Membran 5 aus (vgl. Pfeil in Figur 8 C).

Durch das Anlegen einer Wechselspannung wird das gesamte System zum Schwingen angeregt, wechselt also periodisch zwischen den Extremzuständen entsprechend Figur 8 B und C. Ein Betrieb des Aerosolgenerators 16 findet idealerweise mit einer Anregungsfrequenz der Wechselspannung statt, welche der Resonanzfrequenz und/oder einem Vielfachen der Resonanzfrequenz des Aerosolgenerators 16 entspricht.

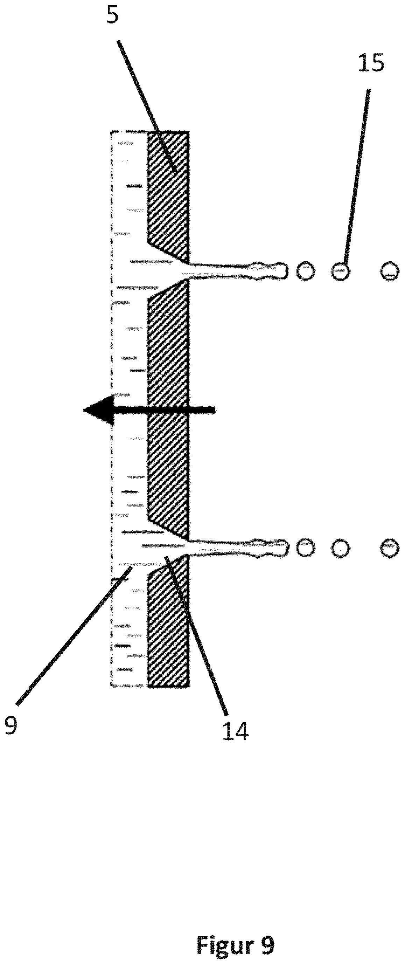

Die Aerosolerzeugung ist beispielhaft in Figur 9 dargestellt. Bewegt sich die Membran 5 aus der Extremposition aus Figur 8 B in Richtung der Extremposition aus Figur 8 C, so drückt die Membran 5 gegen die Flüssigkeit 9, wie in Figur 9 dargestellt. Der Pfeil in Figur 9 beschreibt dabei die Bewegungsrichtung der Membran 5. Dadurch, dass die Membran 5 gegen die Flüssigkeit 9 gedrückt wird, wird die Flüssigkeit 9 zumindest teilweise durch die Öffnungen 14 gedrückt. Dabei reißt die Flüssigkeit 9 auf der Seite der Membran 5, welche der Flüssigkeit 9 abgewandt ist, in Aerosoltropfen 15 ab. Die Größe der Aerosoltropfen wird dabei unter anderem durch den Durchmesser der Öffnungen 14 auf der Flüssigkeit 9 abgewandten Seite der Membran 5 bestimmt.

Die Deformation des Piezoelements 6 ist dabei insbesondere von der angelegten Spannung abhängig. Im Prinzip gilt, in gewissen Grenzen, dass eine höhere Spannung zu einer größeren Deformation führt. Wird der Aerosolgenerator also mit einer Wechselspannung mit höherer Spannung betrieben, sind die Extrempositionen weiter auseinander und es wird eine höhere Menge an Aerosol erzeugt. Durch ein Anpassen der Spannung kann also die Menge an erzeugtem Aerosol angepasst werden. Dabei führt eine Steigerung der Spannung zu einer höheren Auslenkung der Membran 5 und somit zu einer größeren Aerosolerzeugungsrate. Ab einer gewissen Spannung steigert auch die größere Auslenkung der Membran 5 die Aerosolerzeugungsrate nicht mehr. Zudem können in manchen Ausführungsformen bei zu hohen Spannungen mechanische Schädigungen auftreten.

Idealerweise wird der Aerosolgenerator 16 mit einer Sinusfunktion mit einer (Anregungs)Frequenz, die der Resonanzfrequenz des Aerosolgenerators 16 bzw. des Schwingkreises 44 entspricht, angeregt. In manchen Ausführungsformen ist auch ein Betrieb des Aerosolgenerators 16 mit einer von der Resonanzfrequenz abweichenden (Anregungs)Frequenz möglich. In manchen Ausführungsformen ist das System bzw. die Steuerelektronik dazu eingerichtet, die Resonanzfrequenz automatisch nachzusteuern, beispielsweise, wenn sich diese beim Verbrauch der Flüssigkeit 9 verändert.

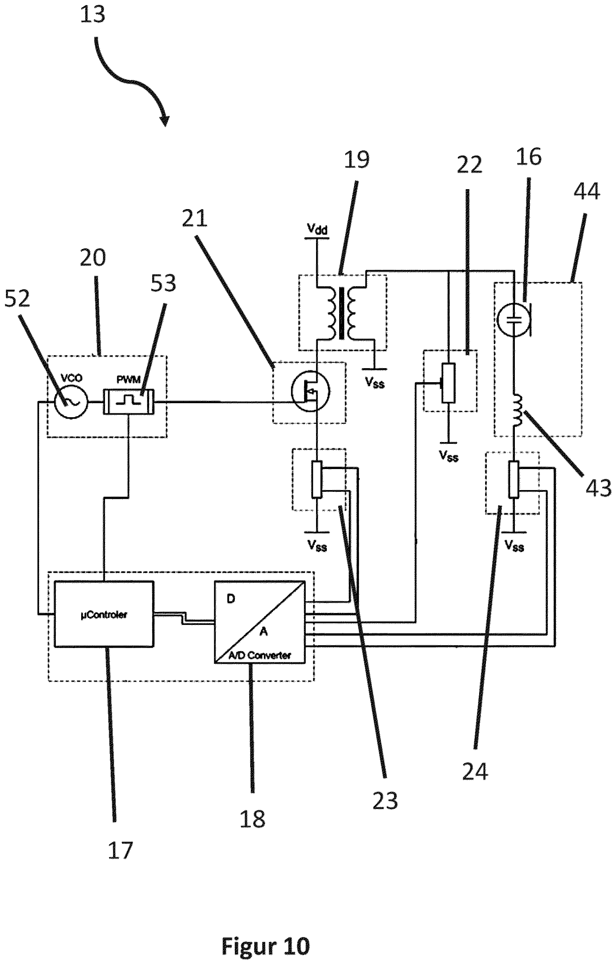

Die Steuerelektronik 13 zur Signalerzeugung, mit welcher der Aerosolgenerator 16 betrieben wird, ist beispielhaft in Figur 10 dargestellt.

Der Aerosolgenerator 16 bildet dabei mit einer in Reihe geschalteten Induktivität 43 einen seriellen Schwingkreis 44. Den Schwingkreis 44 zeichnet dabei aus, dass dieser einen minimalen Durchgangswiderstand aufweist, wenn das elektromechanische System, zum Beispiel bestehend aus Aerosolgenerator 16 und Induktivität 43, mit der Resonanzfrequenz angeregt bzw. betrieben wird. Gleichzeitig entsteht eine Spannungsüberhöhung an der Induktivität 43, beispielsweise eine Spule, und dem Aerosolgenerator 16. Die Spannungsüberhöhung kann beispielhaft ausgenutzt werden um einen erhöhten Wirkungsgrad des Aerosolgenerators zu erreichen.

Beispielsweise kann der Aerosolgenerator 16 mit einer Anregungsfrequenz von 1 kHz bis 200 kHz angesteuert werden. In manchen Ausführungsformen kann auch eine höhere Anregungsfrequenz von bis zu 500 kHz genutzt werden. In manchen Ausführungsformen wird der Aerosolgenerator 16 mit einer Resonanzfrequenz betrieben.

Beispielhaft beträgt die Betriebsspannung am Aerosolgenerator 16 etwa 80 Vss. Die Betriebsspannung wird in der Steuerelektronik 13 durch Speicher- und Schaltelemente eingestellt. In der dargestellten Ausführungsform wird die Betriebsspannung durch einen Transformator 19 zusammen mit einem Transistor 21 eingestellt. Beispielhaft ist der verwendete Transistor ein Feldeffekttransistor, bevorzugt ein Metalloxid-Halbeleiter-Feldeffekttransistor (MOSFET). Es ist alternativ oder ergänzend auch möglich über andere Baugruppen eine entsprechende Einstellung der Betriebsspannung zu erreichen, beispielsweis über eine Schaltung von Iduktivitäten.

Die Resonanzfrequenz wird dabei über den Hochfrequenz-Generator (HF-Generator) 20 eingestellt. Der HF-Generator 20 umfasst dabei zumindest einen Oszillator 52, beispielsweise einen spannungsgesteuerten Oszillator (VCO), und einen Pulsweitenmodulator (PWM) 53. Der HF-Generator 20 wird dabei über einen Mikrocontroller 17 mit, optional integriertem, Digital-Analog-Wandler (A/D-Konverter) 18 angesteuert. Der Pulsweitenmodulator 53 kann alternativ oder ergänzend auch Teil des Mikrocontrollers 17 sein.

Die Resonanzfrequenz wird über die Spannungsmessung mit einen Spannungsmesser 24 am Aerosolgenerator 16 bestimmt. Dazu werden verschiedene Anregungsfrequenzen an dem Aerosolgenerator 16 und/oder dem Schwingkreis 44 vorgegeben und gleichzeitig die Spannung am Spannungsmesser 24 gemessen. Bei der Anregungsfrequenz, bei welcher die höchste Spannung gemessen werden kann, liegt die Resonanzfrequenz. In manchen Ausführungsformen ist die Steuerelektronik 13 dazu eingerichtet, die Resonanzfrequenz automatisch zu ermitteln. Die Resonanzfrequenz des Aerosolgenerators 16 und/oder Schwingkreises 44 ist beispielsweise auch von dem Füllstand der Flüssigkeit 9 in dem Flüssigkeitsreservoir 8 abhängig. Auch eine Abhängigkeit der Resonanzfrequenz von den physikalischen Eigenschaften der Flüssigkeit 9 kann vorliegen. Wird Flüssigkeit 9 verbraucht, so kann sich die Resonanzfrequenz ändern. Die Steuerelektronik 13 ist in manchen Ausführungsformen ebenfalls dazu eingerichtet, Änderungen der Resonanzfrequenz zu erkennen und eine entsprechende Änderung der vorgegebenen Frequenz anzusteuern. Ein Abweichen von der Resonanzfrequenz kann beispielsweise dadurch erkannt werden, dass die durch den Spannungsmesser 24 gemessene Spannung abnimmt.

Es ist möglich, die Resonanzfrequenz eines Aerosolgenerators 16 in einem Frequenzbereich zu ermitteln, indem die Spannung am Aerosolgenerator 16 gemessen wird (Spannungsmesser 24). Verändert man die Anregungsfrequenz durch den gegebenen Frequenzbereich, so ist die Frequenz mit der höchsten Spannung am Aerosolgenerator 16 die Resonanzfrequenz. Übersteigt die Anregungsfrequenz die Resonanzfrequenz fällt die Spannung deutlich ab.

In manchen Ausführungsformen kann die Resonanzfrequenz beispielsweise durch eine Variation der vorgegebenen Anregungsfrequenz um die Resonanzfrequenz während des Betriebs überprüft werden. So wird beispielweise regelmäßig ein Bereich von bis zu 1 kHz rund um die Resonanzfrequenz abgeprüft und die jeweiligen Spannungswerte am Spannungsmesser 24 ermittelt. Die Anregungsfrequenz, bei welcher der höchste Spannungswert ermittelt wird, wird beispielsweise als (neue) Resonanzfrequenz interpretiert und für den Aerosolgenerator 16 vorgegeben.

Neben der Spannungsmessung über den Spannungsmesser 24 wird in der Erfindung auch der Strom an der Primärseite (Strommesser 23) und der Sekundärseite (Strommesser 22) des Transformators 19 gemessen. Über die Messung des Stromes kann eine Aussage über den Wirkungsgrad des Betriebs erfolgen und ein Betrieb bei optimalen Wirkungsgrad kann eingestellt werden. Misst man die Stromstärke sowohl auf der Primärseite als auch auf der Sekundärseite des Transformators 19, so kann man durch Veränderung der Pulsweite 60 des HF-Signals 56 die Spannung mit dem maximalen Wirkungsgrad der Schaltung ermitteln. Der Wirkungsgrad ergibt sich aus dem Verhältnis von Primärleistung zu Sekundärleistung. In manchen Ausführungsformen ist die Steuerelektronik 13 so ausgebildet und eingerichtet, dass automatisch der optimale Wirkungsgrad ermittelt und eingestellt wird.

Durch den HF-Generator 20, insbesondere durch Vorgabe einer Pulsweite durch den Pulsweitenmodulator 53, kann beispielsweise die Amplitude des Anregungssignals 55 eingestellt werden. Bis zu einem bestimmten Punkt kann durch eine größere Pulsweite des HF-Signals 56 der Wirkungsgrad und/oder die erzeugte Aerosolmenge erhöht werden. In manchen Ausführungsformen geht beispielsweise bei 50% und/oder größerer Pulsweite der Transformatorkern in die Sättigung, wodurch sich der Wirkungsgrad verschlechtert. Auch kann ein zu starkes Anregungssignal, beispielsweise durch eine große Pulsweite, zu Schäden an der Membran 5 und/oder der Substratscheibe 7 und/oder dem Piezoelement 6 führen In manchen Ausführungsformen ist bei etwa 30% (+/- 5%) Pulsweite die maximale Aerosolerzeugung bei einem gleichzeitig optimalen Wirkungsgrad erreicht. Die Pulsweite bezieht sich beispielhaft auf die Periode einer Sinuswelle, 50% Pulsweite sind also mit einer halben Periode der Sinuswelle gleichzusetzen.

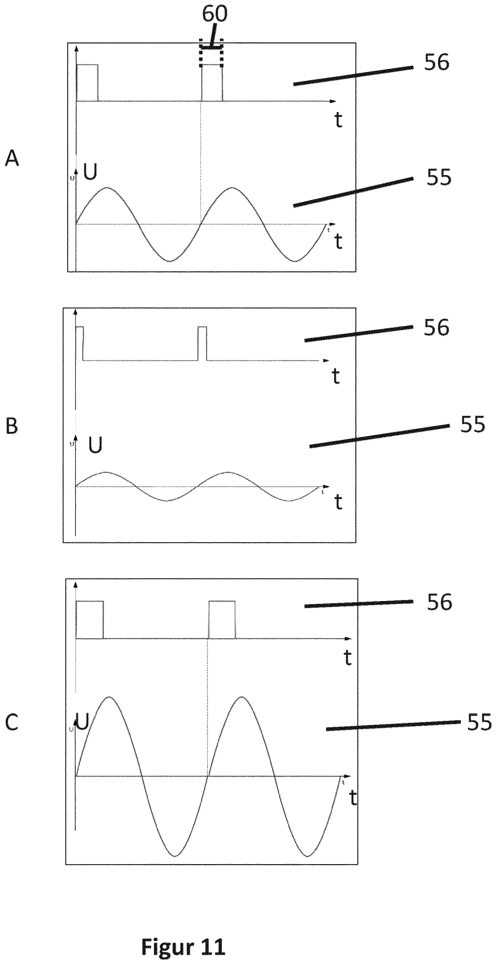

In Figur 11 A-C sind beispielhaft die rechteckigen HF-Signale 56 sowie daraus resultierende Anregungssignale 55 dargestellt. Das rechteckige HF-Signal 56 mit der Pulsweite 60 wird beispielhaft durch den HF-Generator 20 erzeugt. Über dieses HF-Signal 56 wird durch den Transistor 21, den Transformator 19 sowie den Schwingkreis 44 das Anregungssignal 55 erzeugt.

Geringere Pulsweiten 60, also kürzere Rechtecksignale, erzeugen dabei ein Anregungssignal 55 mit geringerer Amplitude bei gleicher Frequenz, wie in Figur 11 B dargestellt. Eine geringere Amplitude führt dabei unter anderem zu einer geringeren Aerosolerzeugung.

Werden größere Pulsweiten 60 erzeugt, so erhöht sich auch die Amplitude des Anregungssignals 55, wobei die Frequenz ebenfalls gleichbleibend ist. Eine höhere Amplitude führt in der Regel auch zu einer höheren Aerosolerzeugung durch den Aerosolgenerator 16.

In manchen Ausführungsformen geht der Transformatorkern bei Pulsweiten 60 ab etwa 50% in eine Sättigung. Dadurch kommt es zu einer Verschlechterung des Wirkungsgrades der Aerosolerzeugung. In manchen Ausführungsformen ist es auch möglich, dass der Transformatorkern erst bei Pulsweiten 60 über 50%, beispielsweise über 60% und mehr, in eine Sättigung geht. Die Pulsweite bezieht sich dabei auf eine Periode der Sinusschwingung. Beispielsweise entspricht also eine Pulsweite 60 von 50% einer halben Periode der Sinusschwingung.

In manchen Ausführungsformen ist eine maximale Aerosolerzeugung bei einem optimalen Wirkungsgrad bei einer Pulsweite 60 von etwa 30% (+/- 5%) erreicht.

Durch eine Modulation der Pulsweite 60 kann die Aerosolerzeugung, insbesondere die Menge des erzeugten Aerosols, gesteuert werden. Wird zu einem Zeitpunkt beispielsweise eine geringe an erzeugtem Aerosol benötigt, so kann eine geringe Pulsweite 60 durch den HF-Generator 20 vorgegeben werden. In manchen Ausführungsformen wird beispielsweise durch das Beatmungsgerät 1 die Menge an zu erzeugendem Aerosol vorgegeben, wobei die Steuerelektronik 13 dazu eingerichtet ist, die Pulsweite 60 entsprechend anzupassen.

Insbesondere kann durch eine Vorgabe und/oder Eingabe durch einen Anwender am Beatmungsgerät 1 und/oder Vernebler 3 die Pulsweite 60 bzw. die zu erzeugende Aerosolmenge angegeben werden. Beispielsweise wird ein Wert für eine zu erzeugende Aerosolmenge am Beatmungsgerät 1 eingestellt und durch den Vernebler 3 in eine entsprechende Pulsweite 60 umgewandelt.

Beispielweise können über das Beatmungsgerät 1 verschiedene Betriebsarten für den Vernebler 3 vorgegeben werden. Eine solche Betriebsart kann beispielsweise so ausgelegt sein, dass der Vernebler 3 immer am optimalen Wirkungsgrad bei maximaler Aerosolerzeugung betrieben wird. Eine weitere Betriebsart kann dadurch gekennzeichnet sein, dass die Aerosolabgabe auf die Atmung des Patienten abgestimmt ist, beispielsweise so, dass lediglich während der Inspiration Aerosol erzeugt wird. Auch kann eine Art Rampe der Pulsweite 60 vorgesehen sein, durch welche die Aerosolabgabe über einen Zeitraum erhöht und/oder verringert wird. In möglichen Ausführungsformen wird beispielsweise die Aerosolerzeugung durch eine sukzessive Erhöhung der Pulsweite 60 des HF-Signals 56 zu Beginn der Inspiration und/oder vor Beginn der Inspiration und/oder zum Ende der Exspiration erhöht. Auch eine Verringerung der Pulsweite 60 zum Ende der Inspiration kann alternativ oder ergänzend vorgesehen sein.

In manchen Ausführungsformen wird die Pulsweite 60 beispielsweise auch an vom Beatmungsgerät 1 gemessene Atemflussraten angepasst. Erhöht sich beispielsweise die Atemflussrate, so wird auch die Pulsweite 60 bzw. die Rate an Aerosolerzeugung erhöht.

In manchen Ausführungsformen ist zudem daran gedacht, die Aerosolerzeugung entsprechend vorgegebener Zeitpunkte zu aktivieren. Beispielsweise kann zu verschiedenen Zeitpunkten eine variierende Menge an Aerosol erzeugt und dem Patienten zugeführt werden. Die Zeitpunkte, zu denen eine Aerosolerzeugung vorgesehen ist, und auch die Menge an zu erzeugendem Aerosol kann zum Beispiel über das Beatmungsgerät 1 programmiert werden. Auch eine automatische Anpassung der Erzeugung von Aerosol, insbesondere der Rate bzw. Menge an erzeugtem Aerosol, und der entsprechenden Veränderung der Pulsweite 60 anhand von physiologischen und/oder medizinischen Daten kann vorgesehen sein.

### Bezugszeichenliste

- 1: Beatmungsgerät
- 2: Schlauch
- 3: Vernebler
- 4: T-Stück
- 5: Membran
- 6: Piezoelement
- 7: Substratscheibe
- 8: Flüssigkeitsreservoir
- 9: Flüssigkeit
- 10: Gehäuse
- 11: Deckel
- 12: elektrische Verbindung
- 13: Steuerelektronik
- 14: Perforation
- 15: Aerosoltropfen
- 16: Aerosolgenerator
- 17: Controller
- 18: A/D-Konverter
- 19: Transformator
- 20: HF-Generator
- 21: Transistor (Feldeffekt, MOSFET)
- 22: Spannungsmesser
- 23: Strommesser
- 24: Strommesser
- 25: Elektronikteil
- 26: Verneblerteil
- 27: Gehäuseteil
- 28: Gehäuseteil
- 29: Dichtungsring
- 30: Platine
- 31: Schraube
- 32: Öffnung
- 33: Kabelanschluss
- 34: Kabel
- 35: Kontaktelement
- 36: Einführöffnung
- 37: Kontaktstifte
- 38: Anschlussstück (Flansch)
- 39: Fassung (Aerosolgenerator)
- 40: Anschlussaufnahme (für Flansch)
- 41: Elektronikkontakt
- 42: Durchführung
- 43: Induktivität
- 44: Schwingkreis
- 45: Maskenanschluss
- 46: Fülleinrichtung
- 47: Füllstandanzeige
- 48: Führungsschiene
- 49: Führungselement
- 50: Führungsnut
- 51: Rastposition
- 52: Oszillator
- 53: Pulsweitenmodulator
- 54: Begrenzung
- 55: Anregungssignal
- 56: HF-Signal
- 57: Öffnung
- 58: Bogenabschnitt
- 59: Wand
- 60: Pulsweite

## Patentansprüche

1. Vernebler (3) zur Erzeugung eines inhalierbaren Tröpfchennebels, Aerosol, umfassend zumindest einen Aerosolgenerator (16), ein Gehäuse (10) mit einem Flüssigkeitsreservoir (8) und eine Steuerelektronik (13), wobei der Aerosolgenerator (16) zumindest aus einer Membran (5), einem Piezoelement (6) und einer Substratscheibe (7) besteht und die Steuerelektronik (13) zumindest einen HF-Generator (20), einen Controller (17), Speicher- und Schaltelemente sowie eine Induktivität (43) umfasst, wobei der Aerosolgenerator (16) zusammen mit der Induktivität (43) einen Schwingkreis (44) bildet und die Steuerelektronik (13) dazu eingerichtet ist, den Aerosolgenerator (16) zumindest teilweise zum Schwingen anzuregen, wobei der Aerosolgenerator (16) so ausgebildet ist, dass durch die Schwingung Aerosoltropfen (15) erzeugbar sind, wobei der HF-Generator (20) ausgebildet ist, um die Menge an erzeugtem Aerosol zu variieren und als HF-Signal (56) Rechtecksignale mit einer Pulsbreite (60) zu erzeugen, **dadurch gekennzeichnet, dass** die Speicher- und Schaltelemente zumindest einen Transformator (19) und einen Transistor (21) umfassen, wobei der Transistor (21) dazu eingerichtet ist, in Verbindung mit dem Transformator (19) aus dem Rechtecksignal und dem Schwingkreis (44) ein Anregungssignal (55) zu erzeugen, wobei die Steuerelektronik (13) ferner zumindest eine Einrichtung zum Messen der Stromstärke an der Primärseite und/oder der Sekundärseite des Transformators (19) umfasst und dazu eingerichtet ist, über die Spannung und die gemessene Stromstärke eine Leistung auf der Primärseite und der Sekundärseite zu ermitteln, über einen Vergleich der ermittelten Leistung der Primärseite mit der ermittelten Leistung der Sekundärseite einen Wirkungsgrad zu bestimmen, auf Basis des Vergleiches einen maximalen Wirkungsgrad zu bestimmen und den maximalen Wirkungsgrad mit der Pulsbreite (60) in Verbindung zu setzen

2. Vernebler (3) nach Anspruch 1, **dadurch gekennzeichnet, dass** die Steuerelektronik (13) dazu eingerichtet ist, eine Anregungsfrequenz für den Aerosolgenerator (16) und/oder den Schwingkreis (44) vorzugeben und ein sinusförmiges Anregungssignal (55) zu erzeugen, wobei das erzeugte Anregungssignal (55) eine höhere Spannung aufweist als das Rechtecksignal.

3. Vernebler (3) nach zumindest einem der vorhergehenden Ansprüche, **dadurch gekennzeichnet, dass** der Vernebler (3) dazu eingerichtet ist, über die Pulsbreite (60) die Menge an erzeugtem Aerosol zu steuern.

4. Vernebler (3) nach zumindest einem der vorhergehenden Ansprüche, **dadurch gekennzeichnet, dass** die Pulsbreite des Rechtecksignals maximal 50 % oder maximal 30 % erreicht

5. Vernebler (3) nach zumindest einem der vorhergehenden Ansprüche, **dadurch gekennzeichnet, dass** der HF-Generator (20) zumindest einen Oszillator (52) und einen Pulsweitenmodulator (53) umfasst, wobei der Pulsweitenmodulator (53) dazu eingerichtet ist, die Pulsbreite (60) des Rechtecksignals vorzugeben.

6. Vernebler (3) nach zumindest einem der vorhergehenden Ansprüche, **dadurch gekennzeichnet, dass** die Steuerelektronik (13) zumindest eine Einrichtung zum Messen einer Spannung umfasst, wobei die Einrichtung zum Messen der Spannung so angeordnet ist, dass die Spannung am Aerosolgenerator aufgenommen wird, und die Steuerelektronik (13) dazu eingerichtet ist, aus der Spannungsmessung die optimale Frequenz des Anregungssignals zu ermitteln.

7. Vernebler (3) nach zumindest einem der vorhergehenden Ansprüche, **dadurch gekennzeichnet, dass** die Steuerelektronik (13) dazu eingerichtet ist, eine optimale Anregungsfrequenz über einen Spannungsabfall am Aerosolgenerator bei Überschreiten der optimalen Anregungsfrequenz zu ermitteln, wobei die optimale Anregungsfrequenz der Resonanzfrequenz des Aerosolgenerators (16) und/oder des Schwingkreises (44) entspricht.

8. Vernebler (3) nach zumindest einem der vorhergehenden Ansprüche, **dadurch gekennzeichnet, dass** die Steuerelektronik (13) dazu eingerichtet ist, eine optimale Anregungsfrequenz und/oder Resonanzfrequenz des Aerosolgenerators (16) und/oder Schwingkreises (44) automatisch zu ermitteln und/oder regelmäßig zu überprüfen und automatisch anzupassen.

9. Vernebler (3) nach zumindest einem der vorhergehenden Ansprüche, **dadurch gekennzeichnet, dass** der Vernebler (3) mit einem Beatmungsgerät (1) verbindbar ist und die Steuerelektronik (13) dazu eingerichtet ist, Vorgaben zur Aerosolerzeugung vom Beatmungsgerät (1) zu empfangen und umzusetzen.

10. Vernebler (3) nach zumindest einem der vorhergehenden Ansprüche, **dadurch gekennzeichnet, dass** der Vernebler (3) ein Verneblerteil (26) und ein Elektronikteil (25) umfasst, wobei das Elektronikteil (25) zumindest die Steuerelektronik (13) umfasst und das Verneblerteil (26) zumindest den Aerosolgenerator (16) und das Flüssigkeitsreservoir (8) umfasst, wobei das Flüssigkeitsreservoir (8) so ausgebildet ist, dass eine Flüssigkeit (9) an die Membran (5) des Aerosolgenerators (16) herangeführt wird.

11. Vernebler (3) nach Anspruch 10, **dadurch gekennzeichnet, dass** der Aerosolgenerator (16) in dem Verneblerteil (26) in einer Fassung (39) gelagert wird, wobei die Fassung (39) über zumindest eine Öffnung (57) verfügt, durch welche die Flüssigkeit (9) an die Membran (5) herangeführt wird, und dass das Flüssigkeitsreservoir (8) über eine Wand (59) von einer Einführöffnung (36) für das Elektronikteil (25) getrennt ist, wobei die Wand (59) mit dem Rand der Öffnung (57) abschließt.

12. Verfahren zur Einstellung der Aerosolgeneration eines Verneblers (3) nach zumindest einem der vorhergehenden Ansprüche, umfassend zumindest einen der folgenden Schritte:
• Finden einer Resonanzfrequenz des Aerosolgenerators (16) und/oder des Schwingkreises (44),
• Bestimmen eines optimalen Wirkungsgrades,
• Einstellen der Pulsbreite,
• Einstellen der Menge an erzeugtem/zu erzeugendem Aerosol.

13. Verfahren nach Anspruch 12, **dadurch gekennzeichnet, dass** zum Finden der Resonanzfrequenz des Aerosolgenerators (16) und/oder des Schwingkreises (44) ein Bereich von Anregungsfrequenzen abgetastet wird und gleichzeitig die Spannung am Aerosolgenerator (16) gemessen wird, wobei die Resonanzfrequenz erreicht ist, wenn eine Erhöhung der Anregungsfrequenz zu einem Abfall der gemessenen Spannung führt.

14. Verfahren nach zumindest einem der Ansprüche 12 und 13, **dadurch gekennzeichnet, dass** das Bestimmen des optimalen Wirkungsgrades eine Messung der Stromstärke an der Primärseite und der Sekundärseite des Transformators umfasst, aus der jeweiligen Stromstärke über die Spannung eine Leistung jeweils für die Primärseite und die Sekundärseite ermittelt wird und ein Wirkungsgrad aus einem Vergleich der ermittelten Leistungen bestimmt wird, wobei das Bestimmen des optimalen Wirkungsgrades weiter eine Variation der Pulsbreite (60) umfasst, wobei durch die Variation der Pulsbreite (60) eine Variation des Wirkungsgrades folgt und der höchste bestimmte Wirkungsgrad als der optimale Wirkungsgrad angesehen wird.

15. Verfahren nach zumindest einem der Ansprüche 12 bis 14, **dadurch gekennzeichnet, dass** das Einstellen der Menge an erzeugtem/zu erzeugendem Aerosol eine Variation der Pulsbreite (60) umfasst, wobei einer Verringerung der Pulsbreite (60) unterhalb der Pulsbreite (60) für den optimalen Wirkungsgrad eine Verringerung der erzeugten Aerosolmenge folgt.

16. Beatmungssystem (61), umfassend zumindest ein Beatmungsgerät (1) und einen Vernebler (3) nach zumindest einem der Ansprüche 1 bis 11, **dadurch gekennzeichnet, dass** das Beatmungssystem (61) zumindest eine Steuerelektronik (13) zur Steuerung des Verneblers (3) umfasst.

17. Beatmungssystem (61) nach Anspruch 16, **dadurch gekennzeichnet, dass** die Steuerelektronik (13) zumindest teilweise in dem Vernebler (3) angeordnet ist.

18. Beatmungssystem (61) nach zumindest einem der Ansprüche 16 und 17, **dadurch gekennzeichnet, dass** das Beatmungsgerät (1) zumindest eine Steuereinheit umfasst, welche dazu eingerichtet ist, Vorgaben zur Aerosolerzeugung an die Steuerelektronik (13) weiterzugeben, wobei die Vorgaben zumindest eine Einstellung zur Menge, Dauer und/oder Zeitpunkt der Aerosolerzeugung umfassen.

## Claims

1. A nebulizer (3) for generating an inhalable mist of droplets, aerosol, comprising at least one aerosol generator (16), a housing (10) with a liquid reservoir (8), and control electronics (13), wherein the aerosol generator (16) consists at least of a membrane (5), a piezo element (6), and a substrate disk (7) and the control electronics (13) comprise at least one HF generator (20), a controller (17), storage and switching elements, and an inductance (43), wherein the aerosol generator (16) together with the inductance (43) forms a resonant circuit (44) and the control electronics (13) are configured to at least partially excite the aerosol generator (16) to vibrate, wherein the aerosol generator (16) is designed so that aerosol drops (15) can be generated by the vibration, wherein the HF generator (20) is designed to vary the amount of generated aerosol and to generate, as an HF signal (56), square-wave signals with a pulse width (60), **characterized in that** the storage and switching elements comprise at least one transformer (19) and a transistor (21), wherein the transistor (21) is configured to generate an excitation signal (55) from the square-wave signal and the resonant circuit (44) in conjunction with the transformer (19), wherein the control electronics (13) also comprise at least one apparatus for measuring the current strength on the primary side and/or the secondary side of the transformer (19) and is configured to ascertain a power on the primary side and the secondary side via the voltage and the measured current strength, to determine an efficiency via a comparison of the ascertained power of the primary side with the ascertained power of the secondary side, to determine a maximum efficiency on the basis of the comparison, and to correlate the maximum efficiency with the pulse width (60).

2. The nebulizer (3) according to claim 1, **characterized in that** the control electronics (13) are configured to specify an excitation frequency for the aerosol generator (16) and/or the resonant circuit (44) and to generate a sinus-shaped excitation signal (55), wherein the generated excitation signal (55) has a higher voltage than the square-wave signal.

3. The nebulizer (3) according to at least one of the preceding claims, **characterized in that** the nebulizer (3) is configured to control the amount of generated aerosol via the pulse width (60).

4. The nebulizer (3) according to at least one of the preceding claims, **characterized in that** the pulse width of the square-wave signal reaches maximally 50% or maximally 30%.

5. The nebulizer (3) according to at least one of the preceding claims, **characterized in that** the HF generator (20) comprises at least one oscillator (52) and a pulse width modulator (53), wherein the pulse width modulator (53) is configured to specify the pulse width (60) of the square-wave signal.

6. The nebulizer (3) according to at least one of the preceding claims, **characterized in that** the control electronics (13) comprise at least one apparatus for measuring a voltage, wherein the apparatus for measuring the voltage is arranged so that the voltage is received at the aerosol generator, and the control electronics (13) are configured to ascertain the optimal frequency of the excitation signal from the voltage measurement.

7. The nebulizer (3) according to at least one of the preceding claims, **characterized in that** the control electronics (13) are configured to ascertain an optimal excitation frequency via a voltage drop at the aerosol generator when the optimal excitation frequency is exceeded, wherein the optimal excitation frequency corresponds to the resonant frequency of the aerosol generator (16) and/or the resonant circuit (44).

8. The nebulizer (3) according to at least one of the preceding claims, **characterized in that** the control electronics (13) are configured to automatically ascertain and/or regularly check and automatically adjust an optimal excitation frequency and/or resonant frequency of the aerosol generator (16) and/or resonant circuit (44).

9. The nebulizer (3) according to at least one of the preceding claims, **characterized in that** the nebulizer (3) can be connected to a ventilator (1) and the control electronics (13) are configured to receive and implement specifications for aerosol generation from the ventilator (1).

10. The nebulizer (3) according to at least one of the preceding claims, **characterized in that** the nebulizer (3) comprises a nebulizer part (26) and an electronics part (25), wherein the electronics part (25) comprises at least the control electronics (13) and the nebulizer part (26) comprises at least the aerosol generator (16) and the liquid reservoir (8), wherein the liquid reservoir (8) is designed so that a liquid (9) is guided to the membrane (5) of the aerosol generator (16).

11. The nebulizer (3) according to claim 10, **characterized in that** the aerosol generator (16) is mounted in a holder (39) in the nebulizer part (26), wherein the holder (39) has at least one opening (57), through which the liquid (9) is guided to the membrane (5), and **in that** the liquid reservoir (8) is separated from an insertion opening (36) for the electronics part (25) by a wall (59), wherein the wall (59) terminates with the edge of the opening (57).

12. A method for adjusting the aerosol generation of a nebulizer (3) according to at least one of the preceding claims, comprising at least one of the following steps:
• Finding a resonant frequency of the aerosol generator (16) and/or the resonant circuit (44),
• Determining an optimal efficiency,
• Adjusting the pulse width,
• Adjusting the amount of aerosol generated/to be generated.

13. The method according to claim 12, **characterized in that**, to find the resonant frequency of the aerosol generator (16) and/or the resonant circuit (44), a range of excitation frequencies are scanned and at the same time the voltage on the aerosol generator (16) is measured, wherein the resonant frequency is reached when an increase in the excitation frequency leads to a drop in the measured voltage.

14. The method according to at least one of claims 12 and 13, **characterized in that** determining the optimal efficiency comprises a measurement of the current strength on the primary side and the secondary side of the transformer, a power for the primary side and the secondary side, respectively, is ascertained in each case from the current strength over the voltage, and an efficiency is determined from a comparison of the ascertained powers, wherein determining the optimal efficiency also comprises a variation of the pulse width (60), wherein a variation of the efficiency follows due to the variation of the pulse width (60) and the highest determined efficiency is considered as the optimal efficiency.

15. The method according to at least one of claims 12 to 14, **characterized in that** adjusting the amount of aerosol generated/to be generated comprises a variation of the pulse width (60), wherein a reduction in the amount of aerosol generated follows a reduction of the pulse width (60) below the pulse width (60) for the optimal efficiency.

16. A ventilation system (61), comprising at least one ventilator (1) and a nebulizer (3) according to at least one of claims 1 to 11, **characterized in that** the ventilation system (61) comprises at least one set of control electronics (13) for controlling the nebulizer (3).

17. The ventilation system (61) according to claim 16, **characterized in that** the control electronics (13) is arranged at least partially in the nebulizer (3).

18. The ventilation system (61) according to at least one of claims 16 and 17, **characterized in that** the ventilator (1) comprises at least one control unit, which is configured to forward specifications for aerosol generation to the control electronics (13), wherein the specifications comprise at least one setting for the amount, duration and/or time of the aerosol generation.

## Revendications

1. Nébuliseur (3) destiné à produire un brouillard de gouttelettes (aérosol) inhalable, comportant au moins un générateur d'aérosol (16), un boîtier (10) comprenant un réservoir de liquide (8) et une électronique de commande (13), dans lequel le générateur d'aérosol (16) est constitué au moins d'une membrane (5), d'un élément piézoélectrique (6) et d'un disque de substrat (7) et l'électronique de commande (13) comporte au moins un générateur HF (20), un contrôleur (17), des éléments de mémoire et de commutation ainsi qu'une inductance (43), dans lequel le générateur d'aérosol (16) forme un circuit d'oscillation (44) conjointement avec l'inductance (43) et l'électronique de commande (13) est configurée pour exciter le générateur d'aérosol (16) au moins partiellement en oscillation, dans lequel le générateur d'aérosol (16) est conçu de telle façon que l'oscillation permet de produire des gouttes d'aérosol (15), dans lequel le générateur HF (20) est conçu pour faire varier la quantité d'aérosol produit et pour produire des signaux rectangulaires en tant que signal HF (56) avec une durée d'impulsion (60), **caractérisé en ce que** les éléments de mémoire et de commutation comportent au moins un transformateur (19) et un transistor (21), dans lequel le transistor (21) est configuré pour produire un signal d'excitation (55) en association avec le transformateur (19) à partir du signal rectangulaire et du circuit d'oscillation (44), dans lequel l'électronique de commande (13) comporte en outre au moins un dispositif de mesure de l'intensité du courant sur le côté primaire et/ou le côté secondaire du transformateur (19) et est configurée pour détecter une puissance sur le côté primaire et le côté secondaire par le biais de la tension et de l'intensité de courant mesurée, pour déterminer un rendement par une comparaison de la puissance détectée du côté primaire avec la puissance détectée du côté secondaire, déterminer un rendement maximal sur la base de la comparaison et mettre en relation le rendement maximal avec la durée d'impulsion (60).

2. Nébuliseur (3) selon la revendication 1, **caractérisé en ce que** l'électronique de commande (13) est configurée pour spécifier une fréquence d'excitation pour le générateur d'aérosol (16) et/ou le circuit d'oscillation (44) et pour produire un signal d'excitation (55) sinusoïdal, dans lequel le signal d'excitation (55) produit présente une tension plus élevée que le signal rectangulaire.

3. Nébuliseur (3) selon l'une au moins des revendications précédentes, **caractérisé en ce que** le nébuliseur (3) est configuré pour commander la quantité d'aérosol produit, par le biais de la durée d'impulsion (60).

4. Nébuliseur (3) selon l'une au moins des revendications précédentes, **caractérisé en ce que** la durée d'impulsion du signal rectangulaire atteint au maximum 50 % ou au maximum 30 %.

5. Nébuliseur (3) selon l'une au moins des revendications précédentes, **caractérisé en ce que** le générateur HF (20) comporte au moins un oscillateur (52) et un modulateur de largeur d'impulsion (53), dans lequel le modulateur de largeur d'impulsion (53) est configuré pour spécifier la durée d'impulsion (60) du signal rectangulaire.

6. Nébuliseur (3) selon l'une au moins des revendications précédentes, **caractérisé en ce que** l'électronique de commande (13) comporte au moins un dispositif de mesure de tension, dans lequel le dispositif de mesure de tension est disposé de telle façon que la tension est absorbée au niveau du générateur d'aérosol, et l'électronique de commande (13) est configurée pour détecter la fréquence optimale du signal d'excitation à partir de la mesure de tension.

7. Nébuliseur (3) selon l'une au moins des revendications précédentes, **caractérisé en ce que** l'électronique de commande (13) est configurée pour détecter une fréquence d'excitation optimale par le biais d'une chute de tension au niveau du générateur d'aérosol lors d'un dépassement de la fréquence d'excitation optimale, dans lequel la fréquence d'excitation optimale correspond à la fréquence de résonance du générateur d'aérosol (16) et/ou du circuit d'oscillation (44).

8. Nébuliseur (3) selon l'une au moins des revendications précédentes, **caractérisé en ce que** l'électronique de commande (13) est configurée pour détecter automatiquement et/ou pour vérifier régulièrement et adapter automatiquement une fréquence d'excitation optimale et/ou une fréquence de résonance du générateur d'aérosol (16) et/ou du circuit d'oscillation (44).

9. Nébuliseur (3) selon l'une au moins des revendications précédentes, **caractérisé en ce que** le nébuliseur (3) peut être raccordé à un appareil respiratoire (1) et l'électronique de commande (13) est configurée pour recevoir et mettre en oeuvre des spécifications de l'appareil respiratoire (1) quant à la production d'aérosol.

10. Nébuliseur (3) selon l'une au moins des revendications précédentes, **caractérisé en ce que** le nébuliseur (3) comporte une partie de nébuliseur (26) et une partie électronique (25), dans lequel la partie électronique (25) comporte au moins l'électronique de commande (13) et la partie de nébuliseur (26) comporte au moins le générateur d'aérosol (16) et le réservoir de liquide (8), dans lequel le réservoir de liquide (8) est conçu de telle façon qu'un liquide (9) est amené vers la membrane (5) du générateur d'aérosol (16).

11. Nébuliseur (3) selon la revendication 10, **caractérisé en ce que** le générateur d'aérosol (16) est installé dans une monture (39) dans la partie de nébuliseur (26), dans lequel la monture (39) dispose d'au moins une ouverture (57) à travers laquelle le liquide (9) est amené vers la membrane (5), et **en ce que** le réservoir de liquide (8) est séparé d'une ouverture d'introduction (36) pour la partie électronique (25) par le biais d'une paroi (59), dans lequel la paroi (59) se termine au niveau du bord de l'ouverture (57).

12. Procédé de réglage de la génération d'aérosol d'un nébuliseur (3) selon l'une au moins des revendications précédentes, comportant l'une au moins des étapes suivantes :
• recherche d'une fréquence de résonance du générateur d'aérosol (16) et/ou du circuit d'oscillation (44),
• détermination d'un rendement optimal,
• réglage de la durée d'impulsion,
• réglage de la quantité d'aérosol produit/à produire,

13. Procédé selon la revendication 12, **caractérisé en ce que** pour la recherche de la fréquence de résonance du générateur d'aérosol (16) et/ou du circuit d'oscillation (44), une plage de fréquences d'excitation est explorée et la tension est mesurée simultanément au niveau du générateur d'aérosol (16), dans lequel la fréquence de résonance est atteinte lorsque l'augmentation de la fréquence d'excitation entraîne une chute de la tension mesurée.

14. Procédé selon l'une au moins des revendications 12 et 13, **caractérisé en ce que** la détermination du rendement optimal comporte une mesure d'intensité du courant sur le côté primaire et le côté secondaire du transformateur, une puissance est détectée respectivement pour le côté primaire et le côté secondaire par le biais de la tension à partir de l'intensité du courant respective et un rendement est déterminé à partir d'une comparaison entre les puissances déterminées, dans lequel la détermination du rendement optimal comporte également une variation de la durée d'impulsion (60), dans lequel la variation de la durée d'impulsion (60) amène une variation du rendement et le rendement déterminé le plus élevé est considéré comme le rendement optimal.

15. Procédé selon l'une au moins des revendications 12 à 14, **caractérisé en ce que** le réglage de la quantité d'aérosol produit/à produire comporte une variation de la durée d'impulsion (60), dans lequel une diminution de la durée d'impulsion (60) en dessous de la durée d'impulsion (60) pour le rendement optimal amène une diminution de la quantité d'aérosol produit.

16. Système respiratoire (61), comportant au moins un appareil respiratoire (1) et un nébuliseur (3) selon l'une au moins des revendications 1 à 11, **caractérisé en ce que** le système respiratoire (61) comporte au moins une électronique de commande (13) pour la commande du nébuliseur (3).

17. Système respiratoire (61) selon la revendication 16, **caractérisé en ce que** l'électronique de commande (13) est disposée au moins partiellement dans le nébuliseur (3).

18. Système respiratoire (61) selon l'une au moins des revendications 16 et 17, **caractérisé en ce que** l'appareil respiratoire (1) comporte au moins une unité de commande, laquelle est configurée pour transmettre des spécifications relatives à la production d'aérosol à l'électronique de commande (13), dans lequel les spécifications comportent au moins un réglage de la quantité, de la durée et/ou du moment de la production d'aérosol.
